(19) Europäisches Patentamt / European Patent Office / Office européen des brevets

(11) **EP 1 554 396 B1**

(12) **EUROPÄISCHE PATENTSCHRIFT**

(45) Veröffentlichungstag und Bekanntmachung des Hinweises auf die Patenterteilung:
**15.06.2011 Patentblatt 2011/24**

(51) Int Cl.:
***C12Q 1/68*** *(2006.01)*

(21) Anmeldenummer: **02762252.1**

(22) Anmeldetag: **26.08.2002**

(86) Internationale Anmeldenummer:
**PCT/DE2002/003122**

(87) Internationale Veröffentlichungsnummer:
**WO 2003/018834 (06.03.2003 Gazette 2003/10)**

(54) **VERDRÄNGUNGSASSAY ZUR DETEKTION VON NUKLEINSÄUREOLIGOMER-HYBRIDISIERUNGSEREIGNISSEN**

DISPLACEMENT ASSAY FOR THE DETECTION OF NUCLEIC ACID OLIGOMER HYBRIDIZATION EVENTS

ESSAI DE DEPLACEMENT DESTINE A LA DETECTION D'HYBRIDATIONS D'OLIGOMERES D'ACIDE NUCLEIQUE

(84) Benannte Vertragsstaaten:
**AT BE BG CH CY CZ DE DK EE ES FI FR GB GR IE IT LI LU MC NL PT SE SK TR**

(30) Priorität: **25.08.2001 DE 10141691**
**06.04.2002 PCT/DE02/01269**

(43) Veröffentlichungstag der Anmeldung:
**20.07.2005 Patentblatt 2005/29**

(73) Patentinhaber: **FRIZ Biochem Gesellschaft für Bioanalytik mbH**
**82061 Neuried (DE)**

(72) Erfinder:
• **HARTWICH, Gerhard**
**80639 München (DE)**
• **FRISCHMANN, Peter**
**82145 München (DE)**
• **HAKER, Ute**
**82194 Gröbenzell (DE)**
• **WIEDER, Herbert**
**81373 München (DE)**

(74) Vertreter: **Graf Glück Habersack Kritzenberger**
**Postfach 10 08 26**
**93008 Regensburg (DE)**

(56) Entgegenhaltungen:
WO-A-01/07665     WO-A-93/21530
WO-A-99/51778     WO-A-99/57319

• **MARRAZZA G ET AL: "Disposable DNA electrochemical sensor for hybridization detection" BIOSENSORS & BIOELECTRONICS, ELSEVIER SCIENCE PUBLISHERS, BARKING, GB, Bd. 14, 1999, Seiten 43-51, XP002113746 ISSN: 0956-5663**
• **WANG J ET AL: "Nucleic-acid immobilization, recognition and detection at chronopotentiometric DNA chips" BIOSENSORS & BIOELECTRONICS, ELSEVIER SCIENCE PUBLISHERS, BARKING, GB, Bd. 12, Nr. 7, 1997, Seiten 587-599, XP002113741 ISSN: 0956-5663**
• **UMEK R M ET AL: "Bioelectronic detection of point mutations using discrimination of the H63D polymorphism of the Hfe gene as a model." MOLECULAR DIAGNOSIS: A JOURNAL DEVOTED TO THE UNDERSTANDING OF HUMAN DISEASE THROUGH THE CLINICAL APPLICATION OF MOLECULAR BIOLOGY. UNITED STATES DEC 2000, Bd. 5, Nr. 4, Dezember 2000 (2000-12), Seiten 321-328, XP008018952 ISSN: 1084-8592**

SEQUENCE LISTING

<110> FRIZ Biochem GmbH

<120> Verdrängungsassay zur Detektion von Nukleinsäureoligomer-Hybridisierungsereignissen

<130> FRI097/02

<150> DE 101 41 691.1
<151> 2001-08-25

<150> PCT/DE02/01269
<151> 2002-04-06

<160> 3

<170> PatentIn version 3.1

<210> 1
<211> 20
<212> DNA
<213> Artificial Sequence

<220>
<223> Artificial

<400> 1

```
tagcggataa cacagtcacc
          20
```

<210> 2
<211> 20
<212> DNA
<213> Artificial Sequence

<220>
<223> Artificial

<400> 2

```
tagcggataa cacagtcacc
          20
```

<210> 3
<211> 20
<212> DNA
<213> Artificial Sequence

<220>
<223> Artificial

<400> 3

```
tagcggataa cacagtcacc
          20
```

## Beschreibung

## Technisches Gebiet

Die vorliegende Erfindung betrifft ein Verfahren zur Detektion von Nukleinsäureoligomer-Hybridisierungsereignissen.

## Stand der Technik

In der Krankheitsdiagnose, bei toxikologischen Testverfahren, in der genetischen Forschung und Entwicklung, sowie auf dem Agrar- und pharmazeutischen Sektor werden Immunoassays und zunehmend auch die Sequenzanalyse von DNA und RNA eingesetzt. Neben den bekannten seriellen Verfahren mit autoradiographischer oder optischer Detektion finden zunehmend parallele Detektionsverfahren mittels Array-Technologie unter Verwendung sogenannter DNA- oder Protein-Chips Anwendung. Auch bei den parallelen Verfahren beruht die eigentliche Detektion entweder auf optischen, radiographischen, massenspektrometrischen oder elektrochemischen Methoden.

Oligonukleotid- oder DNA-Chips können neben der Anwendung zur Sequenzierung auch zur SNP- (Single-Nucleotide-Polymorphism) oder Genexpressions-Analyse verwendet werden, da sie es erlauben, das Aktivitätsniveau einer großen Anzahl individueller aktiver Gene (cDNA oder mRNA) eines spezifischen Zelltyps oder Gewebes parallel zu messen, was mit konventionellen (seriellen) Gendetektionsmethoden nur schwer oder unter großem Aufwand möglich ist. Die Analyse pathologisch veränderter Genaktivitäten wiederum kann zur Aufklärung von Krankheitsmechanismen und zur Identifizierung neuer therapeutischer Angriffspunkte beitragen. Daneben ermöglichen (nur) DNA-Chips sogenannte pharmakogenomische Untersuchungen während der klinischen Entwicklung, durch die die Wirksamkeit und Sicherheit der Medikamente deutlich erhöht werden kann. Bei den pharmakogenomischen Untersuchungen steht die Frage im Vordergrund, welche genetischen Faktoren dafür verantwortlich sind, dass Patienten unterschiedliche Reaktionen auf das gleiche Arzneimittel zeigen. Durch umfangreiche Polymorphismen-Analysen (Basenpaar-Mismatch-Analysen) von Genen, die wichtige Stoffwechsel-Enryme kodieren, können Antworten auf solche Fragen gefunden werden.

Zur Genanalyse auf einem Chip wird auf einer Oberfläche eine Bibliothek bekannter DNA-Sequenzen ("Sonden-Oligonukleotide") in einem geordneten Raster fixiert, so dass die Position jeder individuellen DNA-Sequenz bekannt ist. Existieren in der Untersuchungslösung Fragmente aktiver Gene ("Target-Oligonukleotide"), deren Sequenzen zu bestimmten Sonden-Oligonukleotiden auf dem Chip komplementär sind, so können die Target-Oligonukleotide durch Nachweis der entsprechenden Hybridisierungsereignisse auf dem Chip identifiziert (gelesen) werden.

Protein-Chips, deren Test-Sites statt Sonden-Oligonukleotiden bestimmte Antigen- (oder Antikörper-)Sonden tragen, können in der Proteom-Analyse oder in der Parallelisierung der Diagnostik eingesetzt werden.

Die Verwendung radioaktiver Markierungen bei der DNA-/RNA-Sequenzierung ist mit mehreren Nachteilen verbunden, wie z.B. die aufwändigen, gesetzlich vorgeschriebenen Sicherheitsvorkehrungen beim Umgang mit radioaktiven Materialien. Bei der Fluoreszenz- und massenspektrometrischen Detektion sind die Kosten der apparativen Ausstattung sehr hoch. Die Nachteile der Markierung mit radioaktiven Elementen oder Fluoreszenzfarbstoffen können z.T. umgangen werden, wenn Assoziationsereignisse anhand der damit verbundenen Änderung der elektrochemischen Eigenschaften detektiert werden (vgl. WO 97/46568, WO 99/51778, WO 00/31101, WO 00/42217).

In Bezug auf die DNA-Analyse ist es also wünschenswert und für den Anwender vorteilhaft, wenn die Targets (DNA-Fragment) nicht mit einem Detektionslabel modifiziert werden müssen.

Aus der WO 99/51778 ist ein Verfahren zur Detektion von Nukleinsäureoligomer-Hybridisierungsereignissen bekannt. Dabei werden Interkalatoren verwendet, die sich in doppelsträngige DNA einlagern können. Zunächst erfolgt eine Hybridisierung von Ligat-Nukleinsäureoligomeren mit Nukleinsäureoligomer-Liganden und nachfolgend die Einlagerung eines Interkalators in diese Hybride. Anschließend werden der Interkalator selbst oder sogenannte Mediatoren detektiert.

Ein Verfahren zur Detektion von Nukleinsäureoligomer-Hybridisierungsereignissen wird auch von Wang, J. et al. (Biosensors & Bioelectronics, 1997, 12 (7) 587-599) offenbart. Auch hier erfolgt zunächst eine Hybridisierung von Ligat-Nukleinsäureoligomeren mit Nukleinsäureoligomer-Liganden. Zur eigentlichen Detektion wird eine Base des Nukleinsäureoligomerstrangs oxidiert, nämlich die Base Guanin.

Die WO 93/21530 beschäftigt sich mit einem Verfahren zur Empfindlichkeits- und Selektivitätssteigerung bei Immuno-Assys, Molekül-Rezeptor-, DNA-Komplementär-DNA- und Gast-Wirtsmolekül-Wechselwirkungs-Assays. Dabei werden zunächst markierte Analytmoleküle an immobilisierte Partner gebunden. Unmarkierte Analytmoleküle verdrängen bei Kontakt mit den gebundenen, markierten Partnern ihre markierten Gegenstücke aus der Oberflächenbindung, welche dann durch eine Flüssigkeitsströmung wegtransportiert werden. Die Detektion erfolgt in einer separaten Messzone durch optische oder elektrochemische Methoden.

Die WO 99/57319 beschäftigt sich mit der Detektion von Nukleinsäureoligomer-Hybridisierungsereignissen durch elektrochemische Methoden. Es werden eine Reihe von Elektron-Transfer-Einheiten beschrieben, die kovalent an Nukleinsäureoligomere gebunden vorliegen. Durch die Übertragung von Elektronen von einer Elektron-Transfer-Einheit auf beispielsweise eine Elektrode kann die Anwesenheit des durch Anbindung der Elektron-Transfer-Einheit modifizierten Nukleinsäureoligomers detektiert werden.

Schließlich ist aus der WO 01/07665 A2 ein Verfahren zur Detektion von Nukleinsäureoligomer-Hybridisierungsereignissen bekannt, bei dem an eine leitfähige Oberfläche gebundene Ligat-Nukleinsäureoligomere mit in einer Probe enthaltenen Nukleinsäureoligomer-Liganden hybridisieren. Zum Nachweis der Assoziationsereignisse werden entweder die Nukleinsäureoligomer-Liganden mit einem redox-aktiven Detektionslabel versehen oder es kommen mit einem redoxaktiven Detektionslabel ausgestattete Signal-Nukleinsäureoligomer-Liganden zum Einsatz, die an die Nukleinsäureoligomer-Liganden hybridisieren und auf diese Weise einen aus drei Nukleinsäureoligomeren bestehenden Sandwich-Komplex bilden.

Obwohl es also Detektionsmöglichkeiten für Nukleinsäureoligomer-Hybride gibt, ist der Bedarf an einfachen, kostengünstigen, problemlos durchzuführenden und verlässlichen Detektionsprinzipien vor allem im Bereich der weniger dichten Arrays (low density DNA-Chips mit wenigen bis wenigen hundert Test-sites pro cm$^2$ z.B. für sogenannte POC (Point of Care)- Systeme) hoch.

## Darstellung der Erfindung

Aufgabe der vorliegenden Erfindung ist es deshalb, ein Verfahren zur Detektion von Nukleinsäureoligomer-Hybridisierungsereignissen zu schaffen, welches die Nachteile des Standes der Technik nicht aufweist.

Diese Aufgabe wird durch das Verfahren gemäß unabhängigem Patentanspruch 1 gelöst. Weitere vorteilhafte Details, Aspekte und Ausgestaltungen der vorliegenden Erfindung ergeben sich aus den abhängigen Ansprüche, der Beschreibung, den Figuren und den Beispielen.

Im Rahmen der vorliegenden Erfindung werden die folgenden Abkürzungen und Begriffe benutzt:

| | |
|---|---|
| DNA | Desoxyribonukleinsäure |
| RNA | Ribonukleinsäure |
| PNA | Peptidnukleinsäure (Synthetische DNA oder RNA, bei der die Zucker-Phosphat Einheit durch eine Aminosäure ersetzt ist. Bei Ersatz der Zucker-Phosphat Einheit durch die -NH-(CH$_2$)$_2$- N (COCH$_2$-Base)-CH$_2$CO- Einheit hybridisiert PNA mit DNA.) |
| A | Adenin |
| G | Guanin |
| C | Cytosin |
| T | Thymin |
| U | Uracil |
| Base | A, G, T, C oder U |
| Bp | Basenpaar |
| Nukleinsäure | Wenigstens zwei kovalent verbundene Nukleotide oder wenigstens zwei kovalent verbundene Pyrimidin- (z.B. Cytosin, Thymin oder Uracil) oder Purin-Basen (z.B. Adenin oder Guanin). Der Begriff Nukleinsäure bezieht sich auf ein beliebiges "Rückgrat" der kovalent verbundenen Pyrimidin- oder Purin-Basen, wie z.B. auf das Zucker-Phosphat Rückgrat der DNA, cDNA oder RNA, auf ein Peptid-Rückgrat der PNA oder auf analoge Strukturen (z.B. Phosphoramid-, Thio-Phosphat- oder Dithio-Phosphat-Rückgrat). Wesentliches Merkmal einer Nukleinsäure im Sinne der vorliegenden Erfindung ist es, dass sie natürlich vorkommende cDNA oder RNA sequenzspezifisch binden kann. |
| nt | Nukleotid |
| Nukleotid | Monomerbaustein eines Nukleinsäureoligomers |

| | | |
|---|---|---|
| Nukleinsäureoligomer | Nukleinsäure nicht näher spezifizierter Basenlänge (z.B. Nukleinsäure-Oktamer: Eine Nukleinsäure mit beliebigem Rückgrat, bei der 8 Pyrimidin- oder Purin-Basen kovalent aneinander gebunden sind.). |
| ns-Oligomer | Nukleinsäureoligomer |
| Oligomer | Äquivalent zu Nukleinsäureoligomer. |
| Oligonukleotid | Äquivalent zu Oligomer oder Nukleinsäureoligomer, also z.B. ein DNA-, PNA- oder RNA-Fragment nicht näher spezifizierter Basenlänge. |
| Oligo | Abkürzung für Oligonukleotid. |
| Sequenz | Nukleotidabfolge in einem Nukleinsäureoligomer |
| komplementär | Zur Ausbildung der Watson-Crick Struktur doppelsträngiger Nukleinsäureoligomere assoziieren, i.e. hybridisieren, die beiden Einzelstränge, wobei die Nukleotidabfolge des einen Strangs komplementär zur Nukleotidabfolge des anderen Strangs ist, so dass die Base A (bzw. C) des einen Strangs mit der Base T (bzw. G) des anderen Strangs Wasserstoffbrücken ausbildet (bei RNA ist T durch Uracil ersetzt). |
| Mismatch | Zur Ausbildung der Watson-Crick Struktur doppelsträngiger Nukleinsäureoligomere hybridisieren die beiden Einzelstränge derart, dass die Base A (bzw. C) des einen Strangs mit der Base T (bzw. G) des anderen Strangs Wasserstoffbrücken ausbildet (bei RNA ist T durch Uracil ersetzt). Jede andere Basenpaarung innerhalb des Hybrids bildet keine Wasserstoffbrücken aus, verzerrt die Struktur und wird als "Mismatch" bezeichnet. |
| Perfekter Match | Hybrid aus zwei komplementären Nukleinsäure-Oligomeren, bei dem kein Mismatch auftritt. |
| ss | Single strand (Einzelstrang) |
| ds | Double strand (Doppelstrang) |
| Oxidationsmittel | Chemische Verbindung (chemische Substanz), die durch Aufnahme von Elektronen aus einer anderen chemischen Verbindung (chemischen Substanz) diese andere chemische Verbindung (chemische Substanz) oxidiert. |
| Reduktionsmittel | Chemische Verbindung (chemische Substanz), die durch Abgabe von Elektronen an eine andere chemische Verbindung (chemische Substanz) diese andere chemische Verbindung (chemische Substanz) reduziert. |
| redoxaktiv | Bezeichnet die Eigenschaft einer Einheit unter bestimmten äußeren Umständen an ein geeignetes Oxidationsmittel Elektronen abzugeben oder von einem geeigneten Reduktionsmittel Elektronen aufzunehmen. |
| EDTA | Ethylendiamin-Tetraacetat (Natriumsalz) |
| sulfo-NHS | N-Hydroxysulfosuccinimid |
| NHS | N-Hydroxysuccinimid |
| EDC | (3-Dimethylaminopropyl)-carbodiimid |
| HEPES | N-[2-Hydroxyethyl]piperazin-N'-[2-ethansulfonsäure] |
| Tris | Tris-(hydroxymethyl)-aminomethan |
| Ligand | Bezeichnung für Nukleinsäureoligomer-Liganden, die von Ligat- Nukleinsäureoligomeren spe- |

zifisch gebunden werden.

| | |
|---|---|
| Ligat | Bezeichnung für Ligat-Nukleinsäureoligomere |
| Linker | Molekulare Verbindung zwischen zwei Molekülen bzw. zwischen einem Oberflächenatom, Oberflächenmolekül oder einer Oberflächenmolekülgruppe und einem anderen Molekül. In der Regel sind Linker als Alkyl-, Alkenyl-, Alkinyl-, Hetero-Alkyl-, Hetero-Alkenyl- oder Hetero-Alkinylkette käuflich zu erwerben, wobei die Kette an zwei Stellen mit (gleichen oder verschiedenen) reaktiven Gruppen derivatisiert ist. Diese Gruppen bilden in einfachen/bekannten chemischen Reaktionen mit dem entsprechenden Reaktionspartner eine kovalente chemische Bindung aus. Die reaktiven Gruppen können auch photoaktivierbar sein, d.h. die reaktiven Gruppen werden erst durch Licht bestimmter oder beliebiger Wellenlänge aktiviert. Bevorzugte Linker sind solche der Kettenlänge 1 - 20, insbesondere der Kettenlänge 1 - 14, wobei die Kettenlänge hier die kürzeste durchgehende Verbindung zwischen den zu verbindenden Strukturen, also zwischen den zwei Molekülen bzw. zwischen einem Oberflächenatom, Oberflächenmolekül oder einer Oberflächenmolekülgruppe und einem anderen Molekül, darstellt. |
| Spacer | Äquivalent zu Linker. |
| Mica | Muskovit-Plättchen, Trägermaterial zum Ausbringen dünner Schichten. |
| $Au\text{-}S\text{-}(CH_2)_2\text{-}ss\text{-}oligo$ | Gold-Film auf Mica mit kovalent aufgebrachter Monolayer aus derivatisiertem Einzelstrang-Oligonukleotid. Hierbei ist die endständige Phosphatgruppe des Oligonukleotids am 3' Ende mit $(HO\text{-}(CH_2)_2\text{-}S)_2$ zum $P\text{-}O\text{-}(CH_2)_2\text{-}S\text{-}S\text{-}(CH_2)_2\text{-}OH$ verestert, wobei die S-S Bindung homolytisch gespalten und dadurch je eine Au-S-R Bindung bewirkt wird. |
| $Au\text{-}S\text{-}(CH_2)_2\text{-}ds\text{-}oligo$ | $Au\text{-}S\text{-}(CH_2)_2\text{-}ss\text{-}oligo\text{-}Spacer$ hybridisiert mit dem zu ss-oligo komplementären Oligonukleotid. |
| $K_A$ | Assoziationskonstante für die Assoziation von Ligat und Ligand bzw. Ligat und Signal-Ligand. |
| $E$ | Elektrodenpotential, das an der Arbeitselektrode anliegt. |
| $E_{ox}$ | Potential beim Strom-Maximum der Oxidation einer reversiblen Elektrooxidation oder -reduktion. |
| $i$ | Stromdichte (Strom pro $cm^2$ Elektrodenoberfläche) |
| Cyclovoltametrie | Aufzeichnung einer Strom/Spannungskurve. Hierbei wird das Potential einer stationären Arbeitselektrode zeitabhängig linear verändert, ausgehend von einem Potential, bei dem keine Elektrooxidation oder -reduktion stattfindet bis zu einem Potential, bei dem eine gelöste oder an die Elektrode adsorbierte Spezies oxidiert oder reduziert wird (also Strom fließt). Nach Durchlaufen des Oxidations- bzw. Reduktionsvorgangs, der in der Strom/ Spannungskurve einen zunächst ansteigenden Strom und nach Erreichen eines Maximums einen allmählich abfallenden Strom erzeugt, wird die Richtung des Potentialvorschubs umgekehrt. Im Rücklauf wird dann das Verhalten der Produkte der Elektrooxidation oder -reduktion aufgezeichnet |
| Amperometrie | Aufzeichnung einer Strom/Zeitkurve. Hierbei wird das Potential einer stationären Arbeitselektrode z.B. durch einen Potentialsprung auf ein Potential gesetzt, bei dem die Elektrooxidation oder -reduktion einer gelösten oder adsorbierten Spezies stattfindet und der fließende Strom in Abhängigkeit von der Zeit aufgezeichnet. |
| Chronocoulometrie | Aufzeichnung einer Ladungs/Zeitkurve. Hierbei wird das Potential einer stationären Arbeitselektrode z.B. durch einen Potentialsprung auf ein Potential gesetzt, bei dem die Elektrooxidation oder - reduktion einer gelösten oder adsorbierten Spezies stattfindet und die transferierte Ladung in Abhängigkeit von der Zeit aufgezeichnet Die Chronocoulometrie kann folglich als Integral der Amperometrie verstanden werden. |
| SECM | Scanning Electrochemical Microscopy, Elektrochemische Rastermikroskopie. |

Die vorliegende Erfindung stellt ein Verfahren zur Detektion von Nukleinsäureoligomer-Hybridisierungsereignissen bereit, das die Schritte

a) Bereitstellen einer modifizierten leitfähigen Oberfläche, wobei die Modifikation in der Anbindung von wenigstens zwei Arten von Ligat-Nukleinsäureoligomeren besteht, wobei die verschiedenen Arten von Ligat-Nukleinsäureoligomeren in räumlich abgetrennten Bereichen an die Oberfläche gebunden sind,
b) Bereitstellen von mit einem Detektionslabel modifizierten Signal-Nukleinsäureoligomer-Liganden, wobei als Detektionslabel eine redoxaktive Substanz verwendet wird,
c) Bereitstellen einer Probe mit Nukleinsäureoligomer-Liganden,
d) Inkontaktbringen einer definierten Menge der Signal-Nukleinsäureoligomer-Liganden mit der modifizierten Oberfläche und Inkontaktbringen der Probe und der darin enthaltenen Nukleinsäureoligomer-Liganden mit der modifizierten Oberfläche, wobei zunächst der Schritt

$d_1$) Inkontaktbringen einer definierten Menge der Signal-Nukleinsäureoligomer-Liganden mit der modifizierten Oberfläche,
danach der Schritt
$d_3$) Detektion der Signal-Nukleinsäureoligomer-Liganden und danach der Schritt
$d_2$) Inkontaktbringen der Probe mit der modifizierten Oberfläche durchgeführt wird,

e) Detektion der Signal-Nukleinsäureoligomer-Liganden durch eine oberflächensensitive elektrochemische Methode und
f) Vergleich der in Schritt e) erhaltenen Werte mit den in Schritt $d_3$) erhaltenen Referenzwerten

umfasst.

Da das Inkontaktbringen einer definierten Menge der Signal-Nukleinsäureoligomer-Liganden mit der modifizierten Oberfläche vor dem Inkontaktbringen der Probe mit der modifizierten Oberfläche erfolgt, können die Referenzwerte vor dem Inkontaktbringen der Probe mit der modifizierten Oberfläche jeweils neu bestimmt werden. Dazu wird nach Inkontaktbringen einer definierten Menge der Signal-Nukleinsäureoligomer-Liganden mit der modifizierten Oberfläche eine Detektion der Signal-Nukleinsäureoligomer-Liganden durchgeführt und erst danach die Probe mit der modifizierten Oberfläche in Kontakt gebracht. Danach werden die Signal-Nukleinsäureoligomer-Liganden ein zweites Mal detektiert und die bei der zweiten Detektion bestimmten Werte mit den bei der ersten Detektion bestimmten Referenzwerten verglichen.

Gemäß einer besonders bevorzugten Ausführungsform wird nach der ersten Detektion der Signal-Nukleinsäureoligomer-Liganden die modifizierte Oberfläche gewaschen und nach dem Inkontaktbringen der Probe mit der modifizierten Oberfläche nochmals die gleiche definierte Menge an Signal-Nukleinsäureoligomer-Liganden wie vor der ersten Detektion (Referenzmessung) mit der modifizierten Oberfläche in Kontakt gebracht. Erst danach wird die zweite Detektion der Signal-Nukleinsäureoligomer-Liganden durchgeführt.

Gemäß einer weiteren, besonders bevorzugten Ausführungsform werden nach der ersten Detektion der Signal-Nukleinsäureoligomer-Liganden, vor und/oder während des Waschens der modifizierten Oberfläche, Bedingungen eingestellt oder Maßnahmen ergriffen, die zur zumindest überwiegenden Dissoziation von Ligat-Nukleinsäureoligomeren und Signal-Nukleinsäureoligomer-Liganden führen. Dadurch wird bei dem Wasch-Schritt ein möglichst großer Teil der Signal-Nukleinsäureoligomer-Liganden von der Oberfläche entfernt.

Um die zumindest überwiegende Dissoziation von Ligat-Nukleinsäureoligomeren und Signal-Nukleinsäureoligomer-Liganden zu erreichen kann die Temperatur über die Schmelztemperatur der aus Ligat-Nukleinsäureoligomer und Signal-Nukleinsäureoligomer-Ligand bestehenden doppelsträngigen Oligonukleotide erhöht werden, es können chaotrope Salze zugesetzt werden oder es kann ein Potential angelegt werden, das über dem elektrostringenten Potential liegt.

Alternativ können die Referenzwerte gemäß einer bevorzugten Ausführungsform der vorliegenden Erfindung nach Inkontaktbringen or modifizierten Oberfläche mit Nukleinsäureoligomer-Liganden und Signal-Nukleinsäureoligomer-Liganden und anschließender Detektion der Signal-Nukleinsäureoligomer-Liganden bestimmt werden. Für diese Referenzwertbestimmung werden zunächst Bedingungen eingestellt oder Maßnahmen ergriffen, die zur zumindest überwiegenden Dissoziation von Ligat-Nukleinsäureoligomeren und Nukleinsäureoligomer-Liganden und zur zumindest überwiegenden Dissoziation von Ligat-Nukleinsäureoligomeren und Signal-Nukleinsäureoligomer-Liganden führen, dann wird die modifizierte Oberfläche gewaschen, anschließend werden Signal-Nukleinsäureoligomer-Liganden mit der modifizierten Oberfläche in Kontakt gebracht, wobei die gleiche definierte Menge an Signal-Nukleinsäureoligomer-Liganden wie bei der ersten Zugabe verwendet wird, und schließlich erfolgt eine Detektion der Signal-Nukleinsäureoligomer-Liganden und damit die Bestimmung der Referenzwerte.

Eine dieser im Zusammenhang mit der Dissoziation von Ligat-Nukleinsäureoligomeren und Signal-Nukleinsäureoligomer-Liganden angesprochenen Bedingungen, die auch zu einer erhöhten Dissoziation von Ligat-Nukleinsäureoligomeren und Nukleinsäureoligomer-Liganden führen, ist die Zugabe von chaotropen Salzen, vorteilhafterweise in einer Konzentration von wenigstens 3 mol/l. Auch eine Temperaturerhöhung über die Schmelztemperatur der aus Ligat-Nukleinsäureoligomeren und Nukleinsäureoligomer-Liganden bestehenden doppelsträngigen Oligonukleotide und über die Schmelztemperatur der aus Ligat-Nukleinsäureoligomeren und Signal-Nukleinsäureoligomer-Liganden bestehenden doppelsträngigen Oligonukleotide führt zu einer erhöhten Dissoziation der Doppelstrangoligomere. Bevorzugt wird die Temperatur bis wenigstens 5°C über die Schmelztemperatur erhöht. Als weitere Maßnahme kommt das Anlegen eines Potentials in Frage, das über dem elektrostringenten Potential liegt, insbesondere eine Erhöhung des (negativen) Potentials um wenigstens 10 mV über das elektrostringente Potential, und daneben eine Behandlung mit NaOH.

Die Schmelztemperatur des Doppelstrangs aus Ligat-Nukleinsäureoligomeren und Signal-Nukleinsäureoligomer-Liganden bzw. des Doppelstrangs aus Ligat-Nukleinsäureoligomeren und Nukleinsäureoligomer-Liganden kann entweder bei den entsprechenden äußeren Bedingungen bestimmt werden, also bei gegebener Konzentration der Oligonukleotide, einem bestimmten Salzgehalt, einem bestimmten Salz, in Gegenwart dissoziationsfördernder organischer Lösungsmittel wie Formamid, DMF etc. oder aber auch in weiten Bereichen relativ genau errechnet werden (siehe z.B. den im Internet verfügbaren Oligo-analyzer "www.idtdna.com/program/main/home.asp").

Das Elektrodenpotential kann bei Elektroden-immobilisierten Ligat-Nukleinsäureoligomeren mit assoziierte Gegenstrang dazu benutzt werden, den polyanionischen Gegenstrang von der Elektrode zu verdrängen. Bei negativen bis hin zu leicht positiven Potentialen (E < 0,2 V gegen Silberdraht) liegen sowohl einzel- als auch doppelsträngige, an einer leitfähigen Oberfläche angebundene Nukleinsäure-Oligomere aufgrund der Abstoßung des negativ geladenen DNA-Rückgrats in einer eher gestreckten Konformation vor. Wird das Potential weiter ins Negative verschoben, lädt sich die Oberfläche stärker negativ auf und weniger stark gebundene Nukleinsäureoligomer-Liganden und Signal-Nukleinsäure-Oligomer-Liganden, also solche die im Bindungsbereich einen oder mehrere Basenfehlpaarungen aufweisen, werden von der modifizierten Oberfläche verdrängt. Wie für die Schmelztemperatur beschrieben kann auch dieses sogenannte elektrostringente Potential für bestimmte äußere Parameter experimentell bestimmt werden.

Anionen chaotroper Salze für die Dissoziation von Doppelstrang-Oligonukleotiden sind z.B. $CCl_3COO^-$, $CNS^-$, $CF_3COO^-$, $ClO_4^-$, $I^-$, (siehe auch: Robinson und Grant, Journal of Biological Chemistry, 241, 1966, S. 1329ff und Kessler et al. US 5,753,433). Durch chaotrope Salze wird die Schmelztemperatur erniedrigt.

In sämtlichen, im Rahmen der vorliegenden Erfindung beschriebenen Verfahren kann zur verbesserten Quantifizierung der gemessenen Werte zu jedem Messwert ein Korrektur-Messwert bestimmt werden, der dem Hintergrund-Signal der verwendeten modifizierten Oberfläche entspricht. Der Korrektur-Messwert wird im Allgemeinen von Detektions- und Referenzsignal substrahiert, um die Quantifizierung der Detektion zu verbessern.

Die Bestimmung eines Korrektur-Messwertes für nicht mit Nukleinsäureoligomer-Liganden und Signal-Nukleinsäureoligomer-Liganden assoziierte modifizierte Oberflächen kann im Rahmen der vorliegenden Erfindung grundsätzlich zu drei verschiedenen Zeitpunkten erfolgen: a) nach dem Inkontaktbringen der Probe und der darin enthaltenen Nukleinsäureoligomer-Liganden mit der modifizierten Oberfläche aber vor der Zugabe der Signal-Nukleinsäureoligomer-Liganden; b) vor dem Inkontaktbringen der Probe und der darin enthaltenen Nukleinsäureoligomer-Liganden mit der modifizierten Oberfläche und vor der Zugabe der Signal-Nukleinsäureoligomer-Liganden; c) nach erfolgter Dissoziation der Doppelstranghybride aus Ligat-Nukleinsäureoligomer und Nukleinsäureoligomer-Ligand bzw. aus Ligat-Nukleinsäureoligomer und Signal-Nukleinsäureoligomer-Ligand, nach dem Waschen der modifizierten Oberfläche in Abwesenheit von Signal-Nukleinsäureoligomer-Liganden.

Die Bestimmung der Korrektur-Messwerte kann bei der Durchführung der erfindungsgemäßen Verfahren auch mehrmals zum gleichen oder zu den oben beschriebenen unterschiedlichen Zeitpunkten erfolgen. Der jeweils verwendete Messwert wird dann durch Bildung des arithmetischen Mittels aus den verschiedenen Messungen für den jeweiligen Bereich bestimmt.

Gemäß den nachfolgend geschilderten, besonders bevorzugten Ausführungsformen der vorliegenden Erfindung kann die getrennte Durchführung einer Referenzmessung vermieden werden. Auf der modifizierten Oberfläche sind in diesen Fällen Referenz-Sites aufgebracht, denen nach Zugabe der Probe ein ganz bestimmter Assoziationsgrad zugeordnet werden kann. Das bei der Detektion erhaltene Signal ist dann für diesen bestimmten Grad an Assoziation charakteristisch und kann zur Normierung der Signale der Test-Sites herangezogen werden.

Sämtliche im Rahmen der vorliegenden Erfindung beschriebenen Verfahren werden unter Verwendung einer modifizierten Oberfläche durchgeführt, wobei die Oberfläche durch Anbindung von wenigstens zwei Arten von Ligat-Nukleinsäureoligomeren modifiziert wurde. Die verschiedenen Arten von Ligat-Nukleinsäureoligomeren sind in räumlich im wesentlichen abgetrennten Bereichen an die Oberfläche gebunden. Unter "im wesentlichen abgetrennten Bereichen" werden Bereiche der Oberfläche verstanden, die ganz überwiegend durch Anbindung einer bestimmten Art von Ligat modifiziert sind. Lediglich in Gebieten, in denen zwei solche im wesentlichen abgetrennte Bereiche aneinander grenzen, kann es zu einer räumlichen Vermischung von verschiedenen Arten von Ligat-Nukleinsäureoligomeren kommen. In dem im Rahmen der vorliegenden Erfindung bevorzugten Verfahren erfolgt vor dem Inkontaktbringen der Probe mit der modifizierten Oberfläche der Zusatz von einer Art von Nukleinsäureoligomer-Liganden zu der Probe, wobei der Nukleinsäureoligomer-Ligand ein Bindungspartner mit hoher Assoziationskonstante zu einer bestimmten Art von Ligat-Nukleinsäureoligomeren ist, die in einem bestimmten Bereich (Test-Site $T_{100}$) an die Oberfläche gebunden vorliegt. Diese zugegebene Art von Nukleinsäureoligomer-Ligand assoziiert an die übrigen, an die Oberfläche gebundenen Arten von Ligat-Nukleinsäureoligomeren nicht oder nur sehr geringfügig. Der Nukleinsäureoligomer-Ligand wird dabei in einer Menge zu der Probe zugegeben, die größer ist als die Menge an Nukleinsäureoligomer-Liganden, die notwendig ist, um die Ligat-Nukleinsäureoligomeren der $T_{100}$-Test-Sites vollständig zu assoziieren. Den letzten Schritt dieses Verfahrens bildet - nach Inkontaktbringen der Probe mit der modifizierten Oberfläche, wobei die Probe die Art von Nukleinsäureoligomer-Ligand mit hoher Assoziationskonstante zu den im Bereich $T_{100}$ gebundenen Ligat-Nukleinsäureoligomeren enthält, optionaler stringenter Hybridisierung und nach Inkontaktbringen der Signal-Nukleinsäureoligomer-Liganden mit der modifizierten Oberfläche - der Vergleich der bei der Detektion der Signal-Nukleinsäureoligomer-Liganden erhaltenen Werte mit dem für den Bereich $T_{100}$ erhaltenen Wert. Der für den Bereich $T_{100}$ erhaltene Wert entspricht somit dem Wert bei vollständiger Assoziation (100%).

Gemäß einer besonders bevorzugten Ausführungsform wird eine modifizierte Oberfläche verwendet, die durch Anbindung von wenigstens drei Arten von Ligat-Nukleinsäureoligomeren modifiziert wurde. Die verschiedenen Arten von Ligat-Nukleinsäureoligomeren sind in räumlich im wesentlichen abgetrennten Bereichen an die Oberfläche gebunden. Dabei ist wenigstens eine Art von Ligat-Nukleinsäureoligomeren in einem bestimmten Bereich (Test-Site $T_0$) an die Oberfläche gebunden, von der bekannt ist, dass in der Probe kein Bindungspartner mit hoher Assoziationskonstante enthalten ist, also der komplementäre Nukleinsäureoligomer-Ligand nicht in der Probe vorkommt. Auch in diesem besonders bevorzugten Verfahren erfolgt vor dem Inkontaktbringen der Probe mit der modifizierten Oberfläche der Zusatz von einer Art von Nukleinsäureoligomer-Liganden zu der Probe, wobei die zugegebene Art von Nukleinsäureoligomer-Ligand ein Bindungspartner mit hoher Assoziationskonstante zu einer bestimmten Art von Ligat-Nukleinsäureoligomeren ist, die in einem bestimmten Bereich (Test-Site $T_{100}$) an die Oberfläche gebunden vorliegt. Diese zugegebene Art von Nukleinsäureoligomer-Ligand assoziiert an die übrigen, an die Oberfläche gebundenen Arten von Ligat-Nukleinsäureoligomeren nicht oder nur sehr geringfügig. Der Nukleinsäureoligomer-Ligand wird dabei in einer Menge zu der Probe zugegeben, die größer ist als die Menge an Nukleinsäureoligomer-Liganden, die notwendig ist, um die Ligat-Nukleinsäureoligomeren der $T_{100}$-Test-Sites vollständig zu assoziieren. Den letzten Schritt dieses Verfahrens bildet - nach Inkontaktbringen der Probe mit der modifizierten Oberfläche, wobei die Probe die Art von Nukleinsäureoligomer-Ligand mit hoher Assoziationskonstante zu den im Bereich $T_{100}$ gebundenen Ligat-Nukleinsäureoligomeren enthält, optionaler stringenter Hybridisierung und nach Inkontaktbringen der Signal-Nukleinsäureoligomer-Liganden mit der modifizierten Oberfläche - der Vergleich der bei der Detektion der Signal-Nukleinsäureoligomer-Liganden erhaltenen Werte mit dem für den Bereich $T_{100}$ erhaltenen Wert und mit dem für den Bereich $T_0$ erhaltenen Wert. Der für den Bereich $T_0$ erhaltene Wert entspricht somit dem Wert bei fehlender Assoziation (0%).

Gemäß einer ganz besonders bevorzugten Ausführungsform der beiden oben geschilderten Verfahren, die ohne getrennte Referenz-Messung auskommen, erfolgt vor dem Inkontaktbringen der Probe mit der modifizierten Oberfläche der Zusatz von wenigstens einer weiteren Art von Nukleinsäureoligomer-Ligand zu der Probe, wobei bekannt ist, dass dieser Nukleinsäureoligomer-Ligand in der ursprünglichen Probe nicht enthalten ist. Diese weitere Art von Nukleinsäureoligomer-Ligand weist eine Assoziationskonstante >0 zu einer Art von Ligat-Nukleinsäureoligomeren auf, die in einem bestimmten Bereich (Test-Site $T_n$) an die Oberfläche gebunden vorliegt. Der Nukleinsäureoligomer-Ligand wird in einer solchen Menge zu der Probe gegeben, dass nach dem Inkontaktbringen der Probe mit der modifizierten Oberfläche n% der Ligat-Nukleinsäureoligomere des Test-Sites $T_n$ in assoziierter Form vorliegen. Den letzten Schritt dieses Verfahrens bildet - nach Inkontaktbringen der Probe mit der modifizierten Oberfläche, wobei die Probe die Arten von Nukleinsäureoligomer-Liganden enthält, die an die in den Bereichen $T_{100}$ und $T_n$ gebundenen Ligat-Nukleinsäureoligomeren assoziieren, optionaler stringenter Hybridisierung und nach Inkontaktbringen der Signal-Nukleinsäureoligomer-Liganden mit der modifizierten Oberfläche - der Vergleich der bei der Detektion der Signal-Nukleinsäureoligomer-Liganden erhaltenen Werte mit dem für den Bereich $T_{100}$ erhaltenen Wert, mit dem für den Bereich $T_0$ erhaltenen Wert und mit den für die Bereiche $T_n$ erhaltenen Werte. Der für ein bestimmtes Test-Site $T_n$ erhaltene Wert entspricht somit dem Wert bei Vorliegen von n% Ligat-Ligand-Assoziaten bezogen auf die Gesamtzahl an Ligat-Nukleinsäureoligomeren der entsprechend Art.

Die Menge an Nukleinsäureoligomer-Ligand, die mit der modifizierten Oberfläche in Kontakt gebracht werden muss, um eine n%ige Assoziation am Test-Site $T_n$ zu bewirken, kann vom Fachmann durch einfache Routineuntersuchungen bestimmt werden. Dazu wird z.B. nach Detektion der Werte für $T_0$ und $T_{100}$ eine kalibrierte Messung durchgeführt, bei der die Signalintensität von (unterschiedlichen) Detektionslabel bestimmt wird, mit denen das Ligat-Nukleinsäureoligomer und der Nukleinsäureoligomer-Ligand ausgestattet sind. Das Verhältnis von Ligand-Label-Signal zu Ligat-Label-Signal entspricht n%.

In einer besonders bevorzugten Form der Referenzwertbestimmung über Ermittelung von To- $T_n$- und $T_{100}$-Werten werden als Ligat-Nukleinsäureoligomere zur Modifikation der Messbereiche $T_n$ Mischungen verwendet aus n% Ligat-Nukleinsäureoligomeren, die für das Test-Site $T_{100}$ verwendet werden, und (100-n)% Ligat-Nukleinsäureoligomeren, die für das Test-Site $T_0$ verwendet werden. In diesem Fall kann auf die Ermittlung geeigneter $T_n$-Nukeinsäureoligomer-

Liganden und auf deren Zugabe zur Probe vor Inkontaktbringen der Probe mit der modifizierten Oberfläche verzichtet werden, wenn ausreichend $T_{100}$-Nukleinsäureoligomer-Ligand zur Probe gegeben wird, so dass nicht nur das Test-Site $T_{100}$ vollständig mit $T_{100}$-Nukleinsäureoligomer-Ligand assoziieren kann, sondern auch die jeweiligen n% der $T_n$-Test-Sites mit dem $T_{100}$-Nukleinsäureoligomer-Ligand assoziieren können. Wird eine genügende Anzahl an Referenz-Sites $T_n$ auf der modifizierten Oberfläche aufgebracht, so kann eine Referenzkurve mit hoher Genauigkeit aufgenommen werden. Die Normierung der Messungen der eigentlichen Test-Sites mit Hilfe dieser Referenzkurve verbessert die Reproduzierbarkeit der Analytik mit Hilfe der Chip-Technologie deutlich.

Die Bestimmung der Messwerte für die Bereiche $T_0$, $T_n$ und $T_{100}$ kann bei der Durchführung der erfindungsgemäßen Verfahren auch mehrmals zum gleichen oder zu den oben beschriebenen unterschiedlichen Zeitpunkten erfolgen. Der jeweils verwendete Messwert wird dann durch Bildung des arithmetischen Mittels aus den verschiedenen Messungen für den jeweiligen Bereich bestimmt. Die Messwerte für die Bereiche $T_0$, $T_n$ und $T_{100}$ können, wie vorne beschrieben, durch Ermittelung von Korrektur-Messwerten oder arithmetisch gemittelten Korrektur-Messwerten verbessert werden. In diesem Fall ist es vorteilhaft, auch die Messwerte aller anderen Bereiche der modifizierten Oberfläche (Test-Bereich M) durch Korrektur-Messwerte zu verbessern, wobei mathematische Bestimmungsmethoden bevorzugt werden, die für die Ermittelung der korrigierten Messwerte für die Bereiche $T_0$, $T_n$ und $T_{100}$ und den eigentlichen Messbereich M identisch sind (gleiche Normierung).

Es soll darauf hingewiesen werden, dass das Auffinden eines Nukleinsäureoligomer-Liganden, der nicht in der Probe enthalten ist, keinerlei Probleme bereitet, da auch die umfangreichsten Genome immer noch eine genügende Auswahl an nicht vorhandenen Sequenzen bieten. Für den Fall, dass sich die nicht vorhandene Sequenz von einer anwesenden Sequenz nur durch eine Base unterscheidet, muss der Hybridisierungsschritt unter stringenten Bedingungen durchgeführt werden. Bevorzugt werden aber Sequenzen verwendet, die sich deutlich, also in mehreren Basen, von den in der Probe anwesenden Sequenzen unterscheiden. Besonders gute Ergebnisse werden erzielt, wenn für die Test-Sites und für die Referenz-Sites Oligonukleotide mit der gleichen oder zumindest einer ähnlichen Zahl an Basen verwendet werden.

Die Ligat-Nukleinsäureoligomere der vorliegenden Erfindung bestehen aus n Nukleotiden, die in einer bestimmten Nukleotidabfolge (Sequenz) vorliegen. Die Ligat-Nukleinsäureoligomere sind zu einem Sequenzbereich von n Nukleotiden der Nukleinsäureoligomer-Liganden komplementär oder zumindest überwiegend komplementär. Die Nukleinsäureoligomer-Liganden, deren Anwesenheit anhand der vorliegenden Erfindung detektiert werden soll, können neben dem n Nukleotide langen zusammenhängenden Sequenzbereich, dessen Sequenz komplementär zur n Nukleotid Sequenz der Ligaten ist, weitere Sequenzbereiche aufweisen.

Gemäß einer bevorzugten Ausführungsform der vorliegenden Erfindung werden Ligat-Nukleinsäureoligomere verwendet, wobei jeweils eine Art von Ligat-Nukleinsäureoligomeren aus einer Anzahl von n Nukleotiden einer bestimmten Sequenz besteht. Die Ligat-Nukleinsäureoligomere einer Art sind zu einem Sequenzbereich von n Nukleotiden von Nukleinsäureoligomer-Liganden einer Art komplementär, wobei das Ligat-Nukleinsäureoligomer mit diesem n Nukleotid langen zusammenhängenden Sequenzbereich des Nukleinsäureoligomer-Liganden einen perfekten Match ausbilden kann, d.h. in diesem n Nukleotid langen Bereich bilden Ligat und passender Ligand nach der Assoziation (Hybridisierung) eine Doppelhelix mit n ausschließlich passenden Basenpaarungen. Die an diese Art von Ligat-Nukleinsäureoligomeren assoziierenden Signal-Nukleinsäureoligomer-Liganden weisen dagegen nur teilweise zur n Nukleotid langen Sequenz des Ligaten komplementäre Sequenzbereiche auf, insbesondere besitzen die Signal-Nukleinsäureoligomer-Liganden nur solche Sequenzabschnitte, dass nach Assoziation mit der n Nukleotid langen Sequenz des Ligat-Nukleinsäureoligomers maximal n-1, n-2, n-3, n-4 oder n-5 Nukleotide zueinander komplementär sind.. In diesem Zusammenhang steht n für eine ganze Zahl von 3 bis 80, insbesondere von 5 bis 50, besonders bevorzugt von 15 bis 35 oder von 8 bis 25. Signal-Nukleinsäureoligomer-Liganden, die diese Bedingungen erfüllen, werden im folgenden auch SNP-ID-Liganden genannt.

Gemäß einer besonders bevorzugten Ausführungsform bestehen die Signal-Nukleinsäureoligomer-Liganden, die als SNP-ID-Liganden verwendet werden, aus weniger als n Nukleotiden, wobei alle Nukleotide des Signal-Nukleinsäureoligomer-Liganden sequenzspezifisch komplementär zur n-Nukleotid-Sequenz des Ligat-Nukleinsäureoligomers sind. Alternativ können auch Signal-Nukleinsäureoligomer-Liganden verwendet werden, die aus n oder mehr Nukleotiden bestehen, aber nur solche Bereiche aus n Nukleotiden aufweisen, deren Sequenz in weniger als n Nukleotiden, insbesondere in n-1 bis n-5 Nukleotiden, zur Sequenz des n Nukleotid langen Ligat-Nukleinsäureoligomers komplementär ist. Alternativ können auch Signal-Nukleinsäureoligomer-Liganden verwendet werden, die aus n oder mehr Nukleotiden bestehen, aber nur solche Partialsequenzen aufweisen, dass nach Assoziation von zwei oder mehr Partialsequenzen des Signal-Nukleinsäureoligomer-Liganden an das Ligat-Nukleinsäureoligomer weniger als n Nukleotide des Hybrids aus Ligat und Signal-Nukleinsäureoligomer-Ligand, insbesondere nur n-1 bis n-5 Nukleotide, zueinander komplimentär hybridisiert vorliegen können.

Diese aufeinander abgestimmten komplementären Bereiche von Ligat-Nukleinsäureoligomeren und Signal-Nukleinsäureoligomer-Liganden bringen einen besonderen Vorteil bei der Detektion von Nukleinsäureoligomer-Hybridisierungsereignissen mit sich. Grundsätzlich sollen bei den erfindungsgemäßen Verfahren nur solche Hybridisierungsereignisse detektiert werden, bei denen ein Nukleinsäureoligomer-Ligand an das Ligat-Nukleinsäureoligomer hybridisiert, wobei der Nukleinsäureoligomer-Ligand eine zusammenhängende Nukleotid-Sequenz aufweist, die in allen Basen zu dem Ligat-Nukleinsäureoligomer komplementär ist, womit sich ein sogenannter perfekter Match ausbilden kann.. Um zwischen solchen perfekten Übereinstimmungen und Assoziaten mit Basenfehlpaarungen, sogenannten Mismatches, zu unterscheiden, also Hybridisierungsereignisse zu unterdrücken oder als negativ zu detektieren, bei denen das Assoziat (Hybrid) aus Ligat-Nukleinsäureoligomer und Nukleinsäureoligomer-Ligand ein oder mehrere nicht zueinander komplementäre Basenpaare aufweisen, sind die Assoziationskonstanten (und -zeiten) von perfektem Match, $K_M$, Mismatch, $K_{MM}$, und Hybrid aus Ligat-Nukleinsäureoligomer und Signal-Nukleinsäureoligomer-Ligand, $K_{SIG}$, entscheidend. Die Assoziationskonstante $K_{SIG}$ des Hybrids aus Ligat-Nukleinsäureoligomer und Signal-Nukleinsäureoligomer-Ligand muss nämlich größer sein als die Assoziationskonstante $K_{MM}$ eines Mismatches, da in diesem Fall die Nukleinsäureoligomer-Liganden, deren Assoziation wenigstens einen Mismatch mit den Ligat-Nukleinsäureoligomeren aufweisen, durch die Signal-Nukleinsäureoligomer-Liganden aus ihrer Bindung an die Ligat-Nukleinsäureoligomere verdrängt oder größtenteils verdrängt werden und umgekehrt bereits an das Ligat-Nukleinsäureoligomer assoziierte Signal-Nukleinsäureoligomere nicht mehr oder nur sehr wenig durch Mismatch-Nukleinsäureoligomer-Liganden aus dem Assoziat verdrängt werden können. Gleichzeitig muss aber die Assoziationskonstante $K_{SIG}$ des Hybrids aus Ligat-Nukleinsäureoligomer und Signal-Nukleinsäureoligomer-Ligand kleiner sein als die Assoziationskonstante $K_M$ eines perfekten Matches, da in diesem Fall die Nukleinsäureoligomer-Liganden, deren Assoziation mit den Ligat-Nukleinsäureoligomeren einen perfekten Match ausbilden, durch die Signal-Nukleinsäureoligomer-Liganden nicht aus ihrer Bindung an die Ligat-Nukleinsäureoligomere verdrängt werden und umgekehrt bereits an das Ligat-Nukleinsäureoligomer assoziierte Signal-Nukleinsäureoligomere durch einen Nukleinsäureoligomer-Liganden, der einen perfekten Match ermöglicht, aus dem Assoziat verdrängt werden. Signal-Nukleinsäureoligomer-Liganden, die diese Bedingungen erfüllen, werden als SNP-ID-Liganden bezeichnet. Diese SNP-ID-Liganden besitzen nur solche Sequenzabschnitte, dass nach Assoziation mit dem Ligat-Nukleinsäureoligomer maximal n-1, n-2, n-3, n-4 oder n-5 Nukleotide zueinander komplementär sind.Für die Assoziationskonstanten der möglichen Hybride gilt dann die Korrelation $K_M > K_{SIG} \geq K_{MM}$. Bei geeigneter Wahl des Signal-Nukleinsäureoligomer-Liganden, insbesondere bei Verwendung von Signal-Nukleinsäureoligomer-Liganden, die insgesamt aus weniger als n Nukleotiden bestehen, wobei alle Nukleotide des Signal-Nukleinsäureoligomer-Liganden sequenzspezifisch komplementär zur n-Nukleotid-Sequenz des Ligat-Nukleinsäureoligomers sind, ist die Assoziationskonstante des Hybrids aus Ligat-Nukleinsäureoligomer und Signal-Nukleinsäureoligomer-Ligand etwas größer als die Assoziationskonstante eines Mismatches, da sich die nicht komplementären Basen des Mismatches in der Regel im Inneren der Nukleinsäurekette befinden. Das Hybrid wird dadurch stärker gestört als durch eine fehlende Base am Kettenende, wodurch die Assoziationskonstante des Hybrids aus Ligat-Nukleinsäureoligomer und verkürztem Signal-Nukleinsäureoligomer-Ligand etwas größer ist als die Assoziationskonstante eines Mismatches und es kann somit eine Korrelation $K_M > K_{SIG} > K_{MM}$ eingestellt werden.

In diesem Zusammenhang soll klargestellt werden, dass dem Fachmann bekannt ist, dass Unterschiede im Assoziationsverhalten verschiedener Ligat- / Ligand-Paare aufgrund unterschiedlicher Assoziationskonstanten durch Variation der für die Assoziation zur Verfügung gestellten Konzentrationen an Ligaten und Liganden erhöht oder ausgeglichen werden können beziehungsweise, dass bei Vorliegen ähnlicher Assoziationskonstanten für verschiedenen Ligat- / Ligand-Paare durch Variation der für die Assoziation zur Verfügung gestellten Konzentrationen an Ligaten und Liganden Unterschiede im Assoziationsverhalten verschiedener Ligat- / Ligand-Paare erzeugt werden können. Die oben beschriebenen aufeinander abgestimmten Längen von Ligat-Nukleinsäureoligomeren und Signal-Nukleinsäureoligomer-Liganden bringen einen ganz besonderen Vorteil bei der Detektion von SNPs (Single Nucleotide Polymorphisms) mit sich. SNPs weisen nämlich eine Mutation in genau einer Base auf, die durch das geschilderte Verfahren eindeutig von der nicht-mutierten Variante unterschieden werden kann.

An dieser Stelle soll erwähnt werden, dass die Überlegungen bezüglich der Assoziationskonstanten bei Verwendung von Ligat-Nukleinsäureoligomeren und Signal-Nukleinsäureoligomer-Liganden mit aufeinander abgestimmten Längen auf Verdrängungsassays unter Verwendung von z.B. Ligat-Antikörpern, Signal-Antigen-Liganden und Antigen-Liganden übertragen werden können. An Stelle der Nukleotid-Bereiche für sequenzspezifische Hybridisierung und den damit verbundenen Assoziationskonstanten $K_M$, $K_{SIG}$ und $K_{MM}$ unterscheiden sich in diesem Fall die Assoziationskonstanten der Signal-Antigen/Ligat-Antikörper- und Antigen-Ligand/Ligat-Antikörper-Assoziate z.B. um eine Größenordnung. Im Wesentlichen wird also in den obigen Ausführungen $K_M$, $K_{SIG}$ und $K_{MM}$ durch $A_M$ und $A_{SIG}$ ersetzt, wobei $A_{SIG}$ die Assoziationskonstante der Signal-Antigen/Ligat-Antikörper-Assoziate und $A_M$ die Assoziationskonstanten und Antigen-Ligand/Ligat-Antikörper-Assoziate angibt und $A_M$ z.B. um eine Größenordnung größer ist als $A_{SIG}$, d.h. $A_M = 10 \times A_{SIG}$.

Bei der soeben beschriebenen Verwendung von Ligat-Nukleinsäureoligomeren und Signal-Nukleinsäureoligomer-Liganden mit aufeinander abgestimmten Längen ist zu bedenken, dass die Assoziationskonstante des Hybrids aus Ligat-Nukleinsäureoligomer und Signal-Nukleinsäureoligomer-Ligand und die Assoziationskonstante eines perfekten Matches einen sehr ähnlichen Wert aufweisen. Deswegen wird die folgende Ausführungsform besonders bevorzugt:

Erfolgt zunächst die Zugabe der Nukleinsäureoligomer-Liganden und - nach Entfernen nicht assoziierte Nukleinsäureoligomer-Liganden durch Waschen der modifizierten Oberfläche - getrennt davon die Zugabe der Signal-Nukleinsäureoligomer-Ligandenwird ein molares Verhältnis von Signal-Nukleinsäureoligomer-Liganden zu Ligat-Nukleinsäureoligomeren zwischen 0,01 und 1000, bevorzugt zwischen 0,1 und 100, besonders bevorzugt zwischen 1 und 10 verwendet. Dadurch wird eine Verdrängung der Nukleinsäureoligomere aus dem perfekten Matches durch die Signal-Nukleinsäureoligomer-Liganden vermieden.

Gemäß einer besonders bevorzugten Ausführungsform der vorliegenden Erfindung wird bei der Verwendung von SNP-ID-Liganden als Signal-Nukleinsäureoligomere vor der ersten Detektion der Signal-Nukleinsäureoligomer-Liganden ein Waschen der modifizierten Oberfläche durchgeführt. Dadurch wird die überstehende Lösung mit den darin enthaltenen, nicht an Ligat-Nukleinsäureoligomere assoziierten SNP-ID-Liganden entfernt. Bei der anschließenden Detektion der Signal-Nukleinsäureoligomer-Liganden wird ein Signal gemessen, das ausschließlich von den an die Ligat-Nukleinsäureoligomere gebundenen SNP-ID-Liganden stammt. Die Überlagerung der Messwerte durch ungebundene Signal-Nukleinsäureoligomer-Liganden wird so verhindert, wodurch eine Detektion mit höherer Genauigkeit erreicht wird.

Da wenigstens zwei Arten von Ligat-Nukleinsäureoligomeren an die modifizierte Oberfläche angebunden sind, werden gemäß einer bevorzugten Ausführungsform der vorliegenden Erfindung bei der Verwendung von SNP-ID-Liganden als Signal-Nukleinsäureoligomere auch solche Ligat-Nukleinsäureoligomere verwendet, bei denen die verschiedenen Arten von Ligat-Nukleinsäureoligomeren, insbesondere solche im Messbereich M, jeweils eine unterschiedliche Anzahl $n_{Mi}$ an Nukleotiden aufweisen, wobei $n_{Mi}$ eine ganze Zahl darstellt und die Anzahl der Nukleotide einer Art von Ligat-Nukleinsäureoligomeren angibt, die an der Test-Site i im Messbereich M immobilisiert wurde. Die zu jeder Art von Ligat-Nukleinsäureoligomeren passende Art von SNP-ID-Liganden werden in diesem Fall, wie oben erwähnt, so angepasst, dass sie nur solche Sequenzabschnitte aufweisen, die nach Assoziation mit der $n_{Mi}$ Nukleotid langen Sequenz des Ligat-Nukleinsäureoligomers im Hybrid maximal $n_{Mi}$ -1, $n_{Mi}$ -2, $n_{Mi}$ -3, $n_{Mi}$ -4 oder $n_{Mi}$ -5 zueinander komplementäre Nukleotide aufweisen. Die Variation der Nukleotidlänge der Ligat-Nukleinsäureoligomere bei paralleler Detektion verschiedener Nukleinsäureoligomer-Liganden erlaubt eine gezieltere und erleichterte Auswahl von Ligat-Nukleinsäureoligomeren, die einen perfekten Match mit den Nukleinsäureoligomer-Liganden ermöglichen, wenn die Nukleinsäureoligomer-Liganden der zu untersuchenden Probe einem großen Pool von potentiell vorhandenen Nukleinsäureoligomer-Liganden darstellen, wie dies z. B. bei SNP-Untersuchungen des gesamten menschlichen Genoms der Fall ist. Bei Verwendung von SNP-ID-Liganden muss nämlich nicht mehr darauf geachtet werden, dass die stringenten Hybridisierungsbedingungen, wie sie für SNP-Untersuchungen üblicherweise notwendig sind, um Matches von Mismatches zu unterscheiden, für alle Test-Sites der Paralleluntersuchung auf einer Oberfläche identisch eingestellt werden. Vielmehr ermöglichen die angepassten SNP-ID-Liganden eine indirekte stringente Hybridisierung, die für alle Test-Sites gleichzeitig durchgeführt wird, aber individuell an jedes Test-Site angepasst ist.

Gemäß einer weiteren bevorzugten Ausführungsform der vorliegenden Erfindung werden als Ligat-Nukleinsäureoligomere Ligat-PNA-Oligomere und als Signal-Nukleinsäureoligomer-Liganden Signal-PNA-Oligomer-Liganden verwendet. Der Vorteil der Verwendung von Ligat-PNA-Oligomere und Signal-PNA-Oligomer-Liganden liegt in der Tatsache begründet, dass die Verfahren der vorliegenden Erfindung ohne zusätzliche Modifikation von Ligat-Nukleinsäureoligomeren, Nukleinsäureoligomer-Liganden oder Signal-Nukleinsäureoligomer-Ligand mit detekierbarem Label durchgeführt werden können. PNA unterscheidet sich bekanntermaßen von DNA durch seine fehlende elektrische Ladung. Die Ladung des Nukleinsäureoligomer-Liganden kann bei Verwendung von Ligat-PNA-Oligomeren und Signal-PNA-Oligomer-Liganden als "Label" angesehen werden, das durch geeignete Messungen, insbesondere durch elektrochemische Messungenwie elektrochemische Impedanzmessungen oder durch SECM detektiert werden kann.

Die vorliegende Erfindung stellt somit ein Verfahren zur Detektion von sequenzspezifischen Nukleinsäureoligomer-Hybridisierungsereignissen anhand eines Verdrängungsassays zur Verfügung. Dabei dienen auf Oberflächen immobilisierte Ligat-Nukleinsäureoligomere wie DNA-/RNA-/PNA-Oligomer-Einzelstränge als Assoziationsmatrix (Sonde) zum Nachweis von Targets, also zum Nachweis von Oligonukleotiden oder DNA-Fragmenten. Zunächst werden die Ligat-Nukleinsäureoligomere der Assoziationsmatrix mit einer Lösung von Signal-Nukleinsäureoligomer-Liganden in Kontakt gebracht, wodurch einige der Signal-Nukleinsäureoligomer-Liganden an die oberflächenimmobilisierten Ligat-Nukleinsäureoligomeren hybridisiert werden und die restlichen Signal-Nukleinsäureoligomer-Liganden in der überstehenden Lösung bleiben. Alternativ kann bei der Verwendung von SNP-ID-Liganden als Signal-Nukleinsäureoligomere vor der Detektion der Signal-Nukleinsäureoligomer-Liganden ein Waschen der modifizierten Oberfläche durchgeführt. Dadurch wird die überstehende Lösung mit den darin enthaltenen, nicht an Ligat-Nukleinsäureoligomere assoziierten SNP-ID-Liganden entfernt. Die Signal-Nukleinsäureoligomer-Liganden sind so gewählt, dass die Oberflächen-Assoziate aus Ligat-Nukleinsäureoligomer und Signal-Nukleinsäureoligomer-Ligand eine Assoziationskonstante besitzen, die geringer ist als die Assoziationskonstante zwischen Ligat-Nukleinsäureoligomer und Nukleinsäureoligomer-Ligand. Daneben fungieren die Signal-Nukleinsäureoligomer-Liganden oder die Nukleinsäureoligomer-Liganden entweder selbst als signalgenerierende Substanz für die Detektion oder sie sind mit einer detektierbaren signalgenerierenden Substanz markiert. In einer (Referenz-) Detektion werden die oberflächenimmobilisierten Signal-Nukleinsäureoligomer-Liganden in Abwesenheit von Nukleinsäureoligomer-Liganden - entweder vor Zugabe der Ligand-Nukleinsäureoligomere oder nach Dissoziation der Ligand-Oligonukleotide - mit einer geeigneten Messmethode erfasst. Befinden sich während der Referenzmessung Signal-Nukleinsäureoligomer-Liganden in der überstehenden Lösung, wird als Messmethode eine geeignete oberflächensensitiven Messmethode (z.B. der total internal reflection fluorescence oder elektrochemischen Methoden wie z.B. der Chronocoulometrie) gewählt, die es erlaubt, zwischen oberflächenimmobilisierten Signal-Nukleinsäureoligomer-Liganden und Signal-Nukleinsäureoligomer-Liganden in der Volumen phase zu diskriminieren. In einer weiteren Messung wird - nach Zugabe der die zu untersuchenden Nukleinsäureoligomer-Liganden enthaltenden Lösung zu der modifizierten Oberfläche und Verdrängung eines Teils der Signal-Nukleinsäureoligomer-Liganden durch die Ligat-Nukleinsäureoligomeroder nach Zugabe der Ligand-Oligonukleotide, (stringenter) Hybridisierung, Waschen und Zugabe einer gleichen Menge an Signal-Oligonukleotiden wie bei der Referenzmessung - das im System Ligat-Nukleinsäureoligomer / Signal-Nukleinsäureoligomer-Ligand vorhandene Referenzsignal moduliert und damit eine qualitative wie quantitative Aussagen über Nukleinsäureoligomer-Liganden in der Untersuchungslösung ermöglicht. Die Assoziationsmatrix kann aus nur einem Test-Site mit einer Art von Ligat-Nukleinsäureoligomeren bestehen, bevorzugt wird aber, dass die Assoziationsmatrix aus einer Vielzahl von Test-Sites besteht.

Grundsätzlich erfolgt also die Bestimmung einer unbekannten Art von Nukleinsäureoligomer-Liganden durch Detektion einer dritten Verbindung (Signal-Nukleinsäureoligomer-Ligand), die ebenso wie die unbekannten Art von Nukleinsäureoligomer-Liganden an ein Sonden-Molekül (Ligat-Nukleinsäureoligomer) assoziiert. Bei Anwesenheit von Signal-Nukleinsäureoligomer-Ligand und Nukleinsäureoligomer-Ligand wird durch die stärkere Assoziationsfähigkeit der unbekannten Substanz (Nukleinsäureoligomer-Ligand) zumindest ein Teil der Ligat-Nukleinsäureoligomer besetzt. Ebenso kann natürlich bei Vorliegen von Assoziaten aus Nukleinsäureoligomer-Ligand und Ligat-Nukleinsäureoligomer durch die Zugabe von Signal-Nukleinsäureoligomer-Liganden ein Teil der Nukleinsäureoligomer-Liganden aus dem Komplex Ligand-Ligat verdrängt werden. Dies findet in geringem Maß trotz der stärkeren Assoziationsfähigkeit von Nukleinsäureoligomer-Ligand mit Ligat-Nukleinsäureoligomer statt, da sich in jedem Fall ein Gleichgewicht einstellen wird, welches durch das Verhältnis der Assoziationskonstanten von Nukleinsäureoligomer-Ligand / Ligat-Nukleinsäureoligomer zu Signal-Nukleinsäureoligomer-Ligand / Ligat-Nukleinsäureoligomer und der verwendeten Konzentrationen von Nukleinsäureoligomer-Ligand und Signal-Nukleinsäureoligomer-Ligand bestimmt ist.

Das Verdrängungsassay umfasst also ein Komplexierungsereignis zwischen einem Ligat-Nukleinsäureoligomer und einem Signal-Nukleinsäureoligomer-Liganden, das in Konkurrenz zu einem weiteren Komplexierungsereignis aus eigentlichem Target (Nukleinsäureoligomer-Ligand) und Ligat-Nukleinsäureoligomer steht. Das Verdrängungsassay umfasst insbesondere ein Komplexierungsereignis zwischen einem Ligat-Nukleinsäureoligomer und einem Signal-Nukleinsäureoligomer-Liganden, dem sich ein weiteres Komplexierungsereignis nach Zugabe des eigentlichen Targets (Nukleinsäureoligomer-Ligand) anschließt, welches unter Verdrängung des Signal-Nukleinsäureoligomer-Liganden erfolgt.

**Die Oberfläche**

Mit dem Begriff "Oberfläche" wird jedes Trägermaterial bezeichnet, das geeignet ist direkt oder nach entsprechender chemischer Modifizierung derivatisierte oder nicht-derivatisierte Ligat-Nukleinsäureoligomere kovalent oder über andere spezifische Wechselwirkungen zu binden. Der feste Träger kann aus leitfähigem oder nicht leitfähigem Material bestehen.

Unter dem Begriff "leitfähige Oberfläche" wird jeder Träger mit einer elektrisch leitfähigen Oberfläche beliebiger Dicke verstanden, insbesondere Oberflächen aus Platin, Palladium, Gold, Cadmium, Quecksilber, Nickel, Zink, Kohlenstoff, Silber, Kupfer, Eisen, Blei, Aluminium und Mangan.

Daneben können auch beliebige dotierte oder nicht dotierte Halbleiteroberflächen beliebiger Dicke verwendet werden. Sämtliche Halbleiter können als Reinsubstanzen oder als Gemische Verwendung finden. Als nicht einschränkend gemeinte Beispiele seien an dieser Stelle Kohlenstoff, Silizium, Germanium, $\alpha$-Zinn, Cu(I)- und Ag(I)-Halogenide beliebiger Kristallstruktur genannt. Geeignet sind ebenfalls sämtliche binären Verbindungen beliebiger Zusammensetzung und beliebiger Struktur aus den Elementen der Gruppen 14 und 16, den Elementen der Gruppen 13 und 15, sowie den Elementen der Gruppen 15 und 16. Daneben können ternäre Verbindungen beliebiger Zusammensetzung und beliebiger Struktur aus den Elementen der Gruppen 11, 13 und 16 oder den Elementen der Gruppen 12, 13 und 16 verwendet werden. Die Bezeichnungen der Gruppen des Periodensystems der Elemente beziehen sich auf die IUPAC-Empfehlung von 1985.

**Bindung von Nukleinsäureoligomeren an die Oberfläche**

Als Ligat-Nukleinsäureoligomere werden im Rahmen der vorliegenden Erfindung an eine Oberfläche gebundene Nukleinsäureoligomere bezeichnet. Verfahren zur Immobilisierung von Nukleinsäureoligomeren an einer Oberfläche sind dem Fachmann bekannt. Die Ligat-Nukleinsäureoligomere können z.B. kovalent über Hydroxyl-, Epoxid-, Amino- oder Carboxygruppen des Trägermaterials mit natürlicherweise am Nukleinsäureoligomer vorhandenen oder durch Derivatisierung am Ligat-Nukleinsäureoligomer angebrachten Thiol-, Hydroxy-, Amino- oder Carboxylgruppen an die Oberfläche gebunden werden. Das Ligat-Nukleinsäureoligomer kann direkt oder über einen Linker/Spacer an die Oberflächenatome oder -molekülen einer Oberfläche gebunden werden. Daneben kann das Ligat-Nukleinsäureoligomer durch die bei Immunoassays üblichen Methoden verankert werden wie z.B. durch Verwendung von biotinylierten Ligat-Nukleinsäureoligomeren zur nicht-kovalenten Immobilisierung an Avidin oder Streptavidinmodifizierten Oberflächen. Die chemische Modifikation der Ligat-Nukleinsäureoligomere mit einer Oberflächen-Ankergruppe kann bereits im Verlauf der automatisierten Festphasensynthese oder aber in gesonderten Reaktionsschritten eingeführt werden. Dabei wird auch das Nukleinsäureoligomer direkt oder über einen Linker/Spacer mit den Oberflächenatomen oder -molekülen einer Oberfläche der oben beschriebenen Art verknüpft. Diese Bindung kann auf verschiedene Arten durchgeführt werden (vgl. z.B. WO 00/42217).

**Ligat-Nukleinsäureoligomere / Nukleinsäureoligomer-Liganden / Signal-Nukleinsäureoligomer-Liganden**

Die Ligat-Nukleinsäureoligomere der vorliegenden Erfindung bestehen aus n Nukleotiden, die in einer bestimmten Nukleotidabfolge (Sequenz) vorliegen. Die Ligat-Nukleinsäureoligomere sind zu einem Sequenzbereich von n Nukleotiden der Nukleinsäureoligomer-Liganden komplementär oder zumindest überwiegend komplementär.

Als Nukleinsäureoligomer-Liganden werden Moleküle bezeichnet, die spezifisch mit dem an eine Oberfläche immobilisierten Ligat-Nukleinsäureoligomeren unter Ausbildung eines Doppelstrang-Hybrids wechselwirken. Nukleinsäureoligomer-Liganden im Sinne der vorliegenden Erfindung sind also Nukleinsäureoligomere, die als Komplexbindungspartner des komplementären Nukleinsäureoligomers fungieren. Die Nukleinsäureoligomer-Liganden, deren Vorhandensein anhand der vorliegenden Erfindung detektiert werden soll, können neben dem n Nukleotide langen zusammenhängenden Sequenzbereich, dessen Sequenz komplementär zur n Nukleotid Sequenz der Ligaten ist, weitere Sequenzbereiche aufweisen.

Die Ligat-Nukleinsäureoligomere einer Art sind zu einem Sequenzbereich von n Nukleotiden von Nukleinsäureoligomer-Liganden einer Art komplementär, wobei das Ligat-Nukleinsäureoligomer mit diesem n Nukleotid langen zusammenhängenden Sequenzbereich des Nukleinsäureoligomer-Liganden einen perfekten Match ausbilden kann, d.h. in diesem n Nukleotid langen Bereich bilden Ligat und passender Ligand nach der Assoziation (Hybridisierung) eine Doppelhelix mit n ausschließlich passenden Basenpaarungen.

Die an eine Art von Ligat-Nukleinsäureoligomeren assoziierenden Signal-Nukleinsäureoligomer-Liganden weisen dagegen nur teilweise zur n Nukleotid langen Sequenz des Ligaten komplementäre Sequenzbereiche auf.

In einer besonderen Ausführungsform der vorliegenden Erfindung besitzen die Signal-Nukleinsäureoligomer-Liganden nur solche Sequenzabschnitte, dass nach Assoziation mit der n Nukleotid langen Sequenz des Ligat-Nukleinsäureoligomers maximal n-1, n-2, n-3, n-4 oder n-5 Nukleotide zueinander komplementär sind. In diesem Zusammenhang steht n für eine ganze Zahl von 3 bis 80, insbesondere von 5 bis 50, besonders bevorzugt von 15 bis 35 oder von 8 bis 25. Signal-Nukleinsäureoligomer-Liganden, die diesen Bedingungen genügen, werden auch SNP-ID-Liganden genannt.Die Signal-Nukleinsäureoligomer-Liganden können selbst oder nach entsprechender Modifikation mit einem Detektionslabel durch geeignete Detektionsmethoden bestimmt werden. Die Detektion der Signal-Nukleinsäureoligomer-Liganden erfolgt durch eine oberflächensensitive elektrochemische Detektionsmethode.

Die elektrochemische Detektion kann durch Amperometrie, Chronocoulometrie, Impedanzmessung oder Scanning Electrochemical Microscopy (SECM) erfolgen.

In einer alternativen Ausführungsform der vorliegenden Erfindung werden als Ligat-Nukleinsäureoligomere Ligat-PNA-Oligomere und als Signal-Nukleinsäureoligomer-Liganden Signal-PNA-Oligomer-Liganden verwendet. Der Vorteil der Verwendung von Ligat-PNA-Oligomeren und Signal-PNA-Oligomer-Liganden liegt in der Tatsache begründet, dass ein beliebige Ausführungsform des Verdrängungsassy der vorliegenden Erfindung ohne zusätzliche Modifikation von Ligat-Nukleinsäureoligomer, Nukleinsäureoligomer-Liganden oder Signal-Nukleinsäureoligomer-Ligand mit detekierbarem Label durchgeführt werden kann. PNA unterscheidet sich bekanntermaßen von DNA durch seine fehlende elektrische Ladung. Die Ladung des Nukleinsäureoligomer-Liganden kann bei Verwendung von Ligat-PNA-Oligomere und Signal-PNA-Oligomer-Liganden als "Label" angesehen werden, das durch geeignete Messungen, insbesondere durch elektrochemische Messungen wie elektrochemische Impedanzmessungen oder durch SECM detektiert werden kann.

In einer weiteren alternativen Ausführungsform werden als Signal-Nukleinsäureoligomer-Liganden SNP-ID-Liganden verwendet. Gemäß einer besonders bevorzugten Ausführungsform wird vor der Detektion der Signal-Nukleinsäureoligomer-Liganden ein Waschen der modifizierten Oberfläche durchgeführt. Dadurch wird die überstehende Lösung mit den darin enthaltenen, nicht an Ligat-Nukleinsäureoligomere assoziierten SNP-ID-Liganden entfernt. Bei der anschließenden Detektion der Signal-Nukleinsäureoligomer-Liganden wird ein Signal gemessen, das ausschließlich von den an die Ligat-Nukleinsäureoligomere gebundenen SNP-ID-Liganden stammt. Die Überlagerung der Messwerte durch ungebundene Signal-Nukleinsäureoligomer-Liganden wird so verhindert, wodurch eine Detektion mit höherer Genauigkeit erreicht wird und als Messmethode jede Methode verwendet werden kann, die sensitiv auf das jeweils verwendete Detektins-Label ist.

Im Rahmen der vorliegenden Erfindung besitzen die Signal-Nukleinsäureoligomer-Liganden eine geringere Tendenz zur Komplexbildung mit den Ligat-Nukleinsäureoligomeren als die eigentlichen Nukleinsäureoligomer-Liganden (Targets), d.h. die Assoziationskonstante zwischen Ligand und Ligat ist größer als die Assoziationskonstante zwischen Signal-Ligand und Ligat.

Im Falle eines Ligat-Oligonukleotids mit 20 Basen weist also das Signal-Oligonukleotid nur Nukleinsäure-Bereiche auf, bei denen weniger als 20 konsekutive Basen des Signal-Oligonukleotids komplementär zu den 20 Basen des Ligat-Oligonukleotids sind. Für ein Ligat-Oligonukleotid mit 20 Nukleotiden kann also ein beliebig langes, mit Detektionslabel markiertes Signal-Oligonukleotid verwendet werden, solange das Signal-Oligonukleotid nur Nukleinsäure-Bereiche aufweist, bei denen weniger als 20 konsekutive Basen des Signal-Oligonukleotids komplementär zu den 20 Basen des Ligat-Oligonukleotids sind. Insbesondere kann das Signal-Oligonukleotid ein mit wenigstens einem Detektionslabel markiertes 20 nt-Oligo sein oder eine 20 nt-Oligo-Sequenz enthalten, die komplementär zum 20 nt Ligat-Oligonukleotid ist und ein oder mehrere Basenpaar-Mismatches bei der Komplexierung zwischen Signal-Oligonukleotid und Ligat-Oligonukleotid ausbildet. Es kann auch ein mit wenigstens einem Detektionslabel markiertes Oligonukleotid verwendet werden, das weniger als 20 nt aufweist und vollständig komplementär zum Ligat-Oligonukleotid ist.

Als Nukleinsäureoligomer wird im Rahmen der vorliegenden Erfindung eine Verbindung aus wenigstens zwei kovalent verbundenen Nukleotiden oder aus wenigstens zwei kovalent verbundenen Pyrimidin- (z.B. Cytosin, Thymin oder Uracil) oder Purin-Basen (z.B. Adenin oder Guanin), bevorzugt ein DNA-, RNA- oder PNA-Fragment, verwendet. Der Begriff Nukleinsäure bezieht sich auf ein beliebiges "Rückgrat" der kovalent verbundenen Pyrimidin- oder Purin-Basen, wie z.B. auf das Zucker-Phosphat Rückgrat der DNA, cDNA oder RNA, auf ein Peptid-Rückgrat der PNA oder auf analoge Rückgrat-Strukturen, wie z.B. ein Thio-Phosphat-, ein Dithio-Phosphat- oder ein Phosphoramid-Rückgrat. Wesentliches Merkmal einer Nukleinsäure im Sinne der vorliegenden Erfindung ist die sequenzspezifische Bindung natürlich vorkommender DNA, oder RNA bzw. daraus abgeleitete (transkribierte oder amplifizierte) Strukturen wie cDNA oder amplifizierte cDNA oder amplifizierte RNA (aRNA).

### Detektions-Label / Markierung (Markermolekül)

Signal-Nukleinsäureoligomer-Liganden, die selbst nicht detektiert werden können, werden durch Derivatisierung mit einem oder mehreren detektierbaren Label versehen. Dieses Label ermöglicht die Detektion der Komplexierungsereignisse zwischen dem Signal-Nukleinsäureoligomer-Liganden und dem oberflächengebundenen Ligat-Nukleinsäureoligomer. Das Label kann direkt oder wie im Falle enzym-katalysierter Reaktionen indirekt ein Detektionssignal liefern. Bevorzugte Detektionslabel (Markermoleküle) sind redoxaktive Substanzen.

Bei Verwendung von Ligat-PNA-Oligomeren als Ligat-Nukleinsäureoligomere und Signal-PNA-Oligomer-Liganden als Signal-Nukleinsäureoligomer-liganden kann ohne zusätzliche Modifikation von Ligat-Nukleinsäureoligomer, Nukleinsäureoligomer-Liganden oder Signal-Nukleinsäureoligomer-Liganden mit detekierbarem Label gearbeitet werden. In diesem Fall wird die Ladung des Nukleinsäureoligomer-Liganden als "Label" benutzt, das durch geeignete Messungen, nämlich durch elektrochemische Messungen wie elektrochemische Impedanzmessungen oder durch SECM detektiert werden kann. Zur Detektion durch elektrochemische Methoden werden Redoxmoleküle als Label eingesetzt. Als Redoxlabel können Übergangsemetall-Komplexe, insbesondere solche des Kupfers, Eisens, Rutheniums, Osmiums oder Titans mit Liganden wie Pyridin, 4,7-Dimethylphenanthrolin, 9,10-Phenanthrenquinondiimin, Porphyrine und substituierte Porphyrin-Derivate verwendet werden. Daneben ist der Einsatz von Riboflavin, von Chinonen wie Pyrrollochinolinochinon, Ubichinon, Anthrachinon, Naphtochinon oder Menachinon bzw. Derivaten davon, von Metallocenen und Metallocenderivaten wie Ferrocenen und Ferrocenderivaten, Cobaltocenen und Cobaltocenderivaten, von Porphyrinen, Methylenblau, Daunomycin, Dopamin-Derivaten, Hydrochinon-Derivaten (para- oder ortho-Dihydroxy-Benzol-Derivaten, para- oder ortho-Dihydroxy-Anthrachinon-Derivaten, para- oder ortho-Dihydroxy-Naphtochinon-Derivaten) und ähnlichen Verbindungen möglich.

### Oberflächensensitive Detektionsmethoden

Oberflächensensitive Detektionsmethoden erlauben die Unterscheidung zwischen an eine Oberfläche assoziierten und im Überstand gelösten Markermolekülen. Als Detektionsmethode eigenen sich elektrochemische Verfahren.

Bei elektrochemischen Methoden kann anhand der Kinetik der elektrochemischen Prozesse prinzipiell zwischen an eine Oberfläche adsorbierten und im Überstand gelösten redoxaktiven Detektionslabel unterschieden werden. Oberflächenadsorbierte Detektionslabel werden im allgemeinen schneller elektrochemisch umgesetzt (z.B. oxidiert oder reduziert) als redoxaktive Detektionslabel aus der Volumenphase, da letztere vor der elektrochemischen Umsetzung erst zur (Elektroden-) Oberfläche diffundieren müssen. Als Beispiele für elektrochemische oberflächensensitive Methoden seien die Amperometrie und die Chronocoulometrie genannt.

Die Methode der Chronocoulometrie erlaubt es, oberflächennahe redoxaktive Komponenten von (identischen) redoxaktiven Komponenten in der Volumenphase zu unterscheiden und ist z.B. in Steel, A.B., Herne, T.M. und Tarlov M.J.: Electrochemical Quantitation of DNA Immobilized on Gold, Analytical Chemistry, 1998, Vol. 70, 4670 - 4677 und darin zitierten Literaturstellen beschrieben.

Das Messsignal der Chronocoulometrie (transferierte Ladung Q in Abhängigkeit von der Zeit t) setzt sich aus drei Komponenten zusammen: (i) einem diffusiven Anteil, der durch die gelösten redoxaktiven Komponenten in der Volumenphase hervorgerufen wird und eine $t^{1/2}$-Abhängigkeit aufweist, einen ersten instantanen Anteil, der aus der Ladungsumverteilung in der Doppelschicht (dl, double layer) an der Elektrodenoberfläche resultiert und einen zweiten instantanen Anteil, der durch die Umsetzung redoxaktiver Komponenten bedingt ist, die an der Elektrodenoberfläche adsorbiert (immobilisiert) sind.

Im chronocoulometrischen Experiment ist die Ladung $Q$ als Funktion der Zeit $t$ durch die Cottrell-Gleichung gegeben:

$$Q = \frac{2nFAD_0^{1/2}C_0^{\bullet}}{\pi^{1/2}} t^{1/2} + Q_{dl} + nFA\Gamma_0$$

wobei

$n$:     Anzahl der Elektronen pro Molekül für die Reduktion

$F$:     Faraday Konstante

$A$ :     Elektrodenfläche [cm$^2$]

$D_0$:     Diffusionskoeffizient [cm$^2$/s]

$C_0^{\bullet}$:     Konzentration [mol/cm$^2$]

$Q_{dl}$:     Kapazitive Ladung C

$nFA\Gamma_0$:     Ladungen, die bei der elektrochemischen Umsetzung der adsorbierten redoxaktiven Detektionslabel umgesetzt werden, wobei $\Gamma_0$ [mol/cm$^2$] die Oberflächenbelegungsdichte des Detektionslabels darstellt.

Der Term $\Gamma_0$ steht somit für die Menge an Detektionslabel an der Elektrodenoberfläche. Im chronocoulometrischen Experiment wird bei $t$ = 0 die Summe der Doppelschicht-Ladungen und des Oberflächenüberschusses erhalten.

Ein chronocoulometrisch detektiertes Verdrängungsassay im Sinne der vorliegenden Erfindung soll am Beispiel eines 20 nt Ligat-Nukleinsäureoligomers erläutert werden. Die mit 20 nt Ligat-Oligonukleotiden modifizierte (Arbeits-) Elektrode wird mit einer definierten Menge an Signal-Nukleinsäureoligomer-Liganden, z.B. einem 12 nt Signal-Nukleinsäureoligomer-Liganden, das ein oder mehrere Redoxlabel trägt und zu einem - möglichst oberflächennahen Bereich des Ligat-Oligonukleotids komplementär ist, in Kontakt gebracht, so dass eine Assoziation zwischen Ligat-Oligonukleotid und redox-markiertem ss-Nukleinsäureoligomer-Komplexbildner stattfinden kann. Danach wird die (Arbeits-) Elektrode anfangs auf ein Potential E$_1$ gesetzt, bei dem wenig bis gar keine Elektrolyse (elektrochemische Änderung des Redoxzustands) der Redoxmarkierung stattfinden kann (z.B. bei Ferrocen-modifizierten Ligat-Oligonukleotid ca. 0.1 V gegen Ag/AgCl (sat. KCl)). Dann wird die Arbeitselektrode durch einen Potentialsprung auf ein Potential E$_2$ gesetzt, bei dem die Elektrolyse der Redoxmarkierung im diffusionslimitierten Grenzfall stattfindet (z.B. bei Ferrocen-modifizierten ss-Nukleinsäureoligomer-Komplexbildner ca. 0.5 V gegen Ag/AgCl (sat. KCl)). Die transferierten Ladungen werden in Abhängigkeit von der Zeit aufgezeichnet.

Anschließend wird die Probenlösung zugegeben, die das Ligand-Nukleinsäureoligomer (Target) enthalten soll (kann), welches eine nt-Sequenz aufweist, die in einem Bereich zu den 20 nt der Ligat-Oligonukleotide komplementär ist. Nach Hybridisierung des Targets an die Ligat-Oligonukleotide und somit nach partieller Verdrängung der Signal-Nukleinsäureoligomer-Liganden wird eine zweite elektrochemische Messung durchgeführt. Die Änderung des instantanen Ladungssignals ist proportional zur Anzahl der verdrängten Signal-Oligonukleotid-Liganden und ist somit proportional zur Anzahl der in der Untersuchungslösung vorhandenen Target-Oligonukleotide.

Diese Änderung des instantanen Ladungssignals hängt von der Länge der Ligand-Oligonukleotide, also von der Anzahl der Nukleotide der Ligand-Oligonukleotide ab. Weisen die Ligand-Oligonukleotide eine Länge auf, die ungefähr der Länge der Ligat-Oligonukleotide entspricht oder kürzer als diese ist, so wird eine Abnahme des instantanen Ladungssignals beobachtet, da Signal-Oligonukleotide durch die Ligand-Oligonukleotide aus dem Assoziat mit den Ligat-Oligonukleotiden und damit aus der Nähe der modifizierten Oberfläche verdrängt werden. Weisen die Ligand-Oligonukleotide eine größere Länge als die Ligat-Oligonukleotide auf, so kann nur ein Teil der Nukleotide der Ligand-Oligonukleotide an die Nukleotide der Ligat-Oligonukleotide anbinden und es verbleibt ein überstehender Teil des Ligand-Oligonukleotids mit frei zugänglichen Nukleotiden. In der Regel werden dann die Signal-Oligonukleotide nach Hybridisierung der Ligand-Oligonukleotide an die Ligat-Oligonukleotide an diese freien Basen der Ligand-Oligonukleotide anbinden. Befindet sich der überstehende Teil der Ligand-Oligonukleotide in der Nähe der Oberfläche, so kann der Fall eintreten, dass eine Zunahme des instantanen Ladungssignals beobachtet wird, da sich die Anzahl an Signal-Oligonukleotiden, die sich in der Nähe der Oberfläche befinden durch Zugabe der langkettigen Ligand-Oligonukleotide und Bindung der Signal-Oligonukleotide an diese Ligand-Oligonukleotide vergrößert. Bei sehr langkettigen Ligand-Oligonukleotiden kann sich eine deutlich größere Differenz (Zunahme) zwischen instantanem Ladungssignal vor Ligand-Oligonukleotid-Zugabe und instantanem Landungssignal nach Ligand-Oligonukleotid-Zugabe ergeben als bei kurzkettigen Ligand-Oligonukleotiden (Abnahme des instantanen Landungssignal). Unsichere Ergebnisse werden nur dann erhalten, wenn die Anzahl an Signal-Nukleinsäureoligomer-Liganden, die aus dem Assoziat Signal-Ligand-Oligonukleotid/Ligat-Oligonukleotid verdrängt werden, ungefähr der Anzahl an Signal-Nukleinsäureoligomer-Liganden entspricht, die anschließend an den sich in der Nähe der Oberfläche befindenden, über den an das Ligat-Oligonukleotid assoziierten Teil hinausstehenden Teil der Ligand-Oligonukleotide anbinden.

## Kurze Beschreibung der Zeichnungen

Die Erfindung soll nachfolgend anhand von Ausführungsbeispielen im Zusammenhang mit den Zeichnungen näher erläutert werden. Es zeigen

Fig. 1     eine schematische Darstellung der Detektion von Nukleinsäureoligomer- Hybridisierungsereignissen mittels Verdrängungsassay;

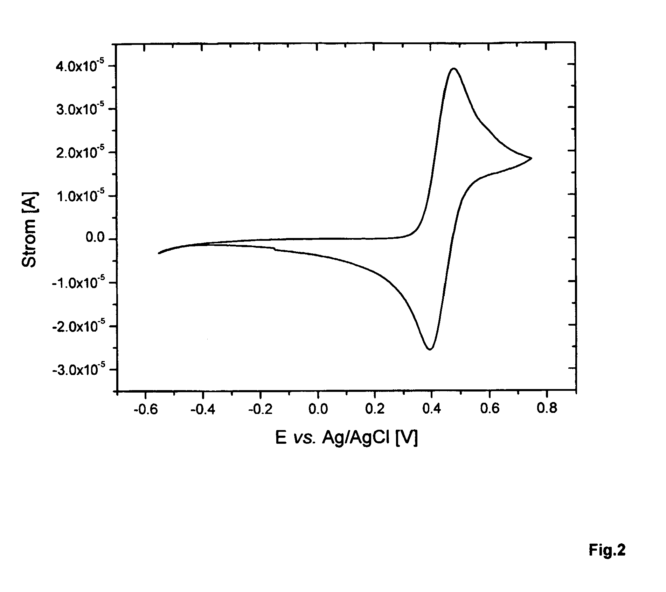

Fig. 2     ein Cyclovoltagramm von Ferrocencarbonsäure (Gold-Arbeitselektrode, Platin- Gegenelektrode, Ag/AgCl (sat. KCl)-Referenzelektrode, 10mM Ferrocencarbonsäure);

Fig. 3     eine chronocoulometrische Messung der sequenzspezifischen Hybridisierung eines 20mer-Ligat-Nukleinsäureoligomeren mit komplementärem Gegenstrang (Nukleinsäureoligomer-Ligand) durch Detektion der durch die Hybridisierung verdrängten ferrocenmarkierten Tetramer-Signal-Nukleinsäureoligomer-Liganden.

## Bezugszeichenliste

A:       Array mit über geeignete Linker x immobilisierten Ligat- Nukleinsäureoligomeren (zwei Test-Sites)
B:       Array inkubiert mit einer Lösung von Signal-Nukleinsäureoligomer-Liganden
C:       Array mit an Ligat-Nukleinsäureoligomeren assoziierten Signal- Nukleinsäureoligomer-Liganden
D:       Array mit spezifischer Komplexierung eines Test-Sites mit Target
E:       Array mit spezifischer Komplexierung eines Ligat-Nukleinsäureoligomeren mit Target und mit an einen Ligat-Nukleinsäureoligomeren assoziierten Signal-Nukleinsäureoligomer-Liganden
101:     Ligat-Nukleinsäureoligomer 1
102:     Ligat-Nukleinsäureoligomer 2
103:     Signal-Nukleinsäureoligomer-Ligand
104:     Detektionslabel, z.B. Ferrocen
105:     Oberfläche, z.B. Gold
106:     Nukleinsäureoligomer-Ligand
①:       Zugabe eines Signal-Nukleinsäureoligomer-Liganden
②:       Hybridisierung
③:       Dissoziation und Entfernen der Signal-Nukleinsäureoligomer-Liganden und anschließende Zugabe des Nukleinsäureoligomer-Liganden
④:       Zugabe des Nukleinsäureoligomer-Liganden
⑤:       Zugabe der Signal-Nukleinsäureoligomer-Liganden

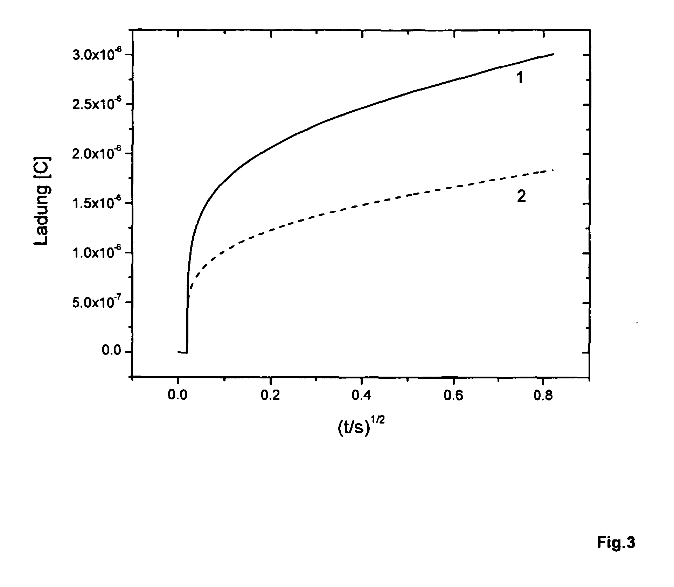

In Figur 3 gibt die mit "1" bezeichnete Kurve die chronocoulometrische Messung nach Hybridisierung der ferrocenmarkierten Tetramer-Signal-Nukleinsäureoligomer-Liganden wieder, während die mit "2" bezeichnete Kurve die chronocoulometrische Messung nach Zugabe des komplementären Nukleinsäureoligomer-Liganden und (partieller) Verdrängung der redoxmarkierten Tetramere von der Ligat-Oberfläche zeigt.

**Wege zur Ausführung der Erfindung**

**Verfahrensvarianten des Verdrängungsassays zur Detektion von Nukleinsäureoligomer-Hybridisierungsereignissen**

Um die Vorteile der DNA-Chip-Technologie auf die Detektion von Nukleinsäureoligomer-Hybriden durch den Verdrängungsassay anzuwenden, werden verschiedene modifizierte Ligat-Nukleinsäureoligomere unterschiedlicher Sequenz mit den oben beschriebenen Immobilisierungstechniken an einen Träger gebunden. Mit der Anordnung der Ligat-Nukleinsäureoligomere bekannter Sequenz an definierten Positionen der Oberfläche, einem DNA-Array, soll das Hybridisierungsereignis eines beliebigen Nukleinsäureoligomer-Liganden oder einer (fragmentierten) Ligand-DNA detektierbar sein, um z.B. Mutationen im Nukleinsäureoligomer-Liganden aufzuspüren und sequenzspezifisch nachzuweisen. Dazu werden auf einer Oberfläche die Oberflächenatome oder -moleküle eines definierten Bereichs (einer Test-Site) mit DNA-/RNA-/PNA-Nukleinsäureoligomeren bekannter, aber beliebiger Sequenz, wie oben beschrieben, verknüpft. Der DNA-Chip kann auch mit einem einzigen Ligat-Oligonukleotid derivatisiert werden. Als Ligat-Nukleinsäureoligomere werden Nukleinsäureoligomere (z.B. DNA-, RNA- oder PNA-Fragmente) der Basenlänge 3 bis 80 oder 3 bis 70 oder 3 bis 50, bevorzugt der Länge 5 bis 50 oder 8 bis 50 oder 5 bis 30, besonders bevorzugt der Länge 15 bis 35 oder 10 bis 30 oder 8 bis 25 verwendet.

Die so bereitgestellte Oberfläche mit immobilisierten Ligat-Oligonukleotiden wird mit einer Lösung einer bestimmten Menge von Signal-Nukleinsäureoligomer-Liganden, z.B. mit Redox-Label markierten Nukleinsäureoligomeren inkubiert, die nur zu einem bestimmten Sequenzabschnitt des Ligat-Oligonukleotids hybridisierbar sind, nicht aber zur gesamten Sequenz des Ligat-Oligonukleotids. Dabei kommt es zur Ausbildung von Hybriden aus Ligat-Nukleinsäureoligomer und den Signal-Nukleinsäureoligomer-Liganden im Bereich komplementärer Sequenzen.

Nach der Hybridisierung zwischen Ligat und Signal-Ligand wird in einer Referenzmessung der oberflächenimmobilisierte Anteil der Signal-Nukleinsäureoligomer-Liganden bestimmt (z.B. durch eine erste chronocoulometrische Messung, vgl. Abschnitt "Oberflächensensitive Detektionsmethoden").

Im nächsten Schritt wird die (möglichst konzentrierte) Untersuchungslösung mit Ligand-Oligonukleotid(en) zur Oberfläche mit immobilisierten Ligat-Oligonukleotiden, assoziierten Signal-Nukleinsäureoligomer-Liganden und überstehender Lösung (mit freien, nicht oberflächenadsorbierten Signal-Nukleinsäureoligomer-Liganden) gegeben. Dabei kommt es nur in dem Fall zur Hybridisierung, in dem die Lösung Ligand-Nukleinsäureoligomer-Stränge enthält, die zu den an die Oberfläche gebundenen Ligat-Nukleinsäureoligomeren komplementär, oder zumindest in weiten Bereichen (bzw. weiteren Bereichen als das Signal-Oligonukleotid) komplementär sind. Die ursprünglich assoziierten Signal-Oligonukleotide werden, zumindest teilweise, verdrängt.

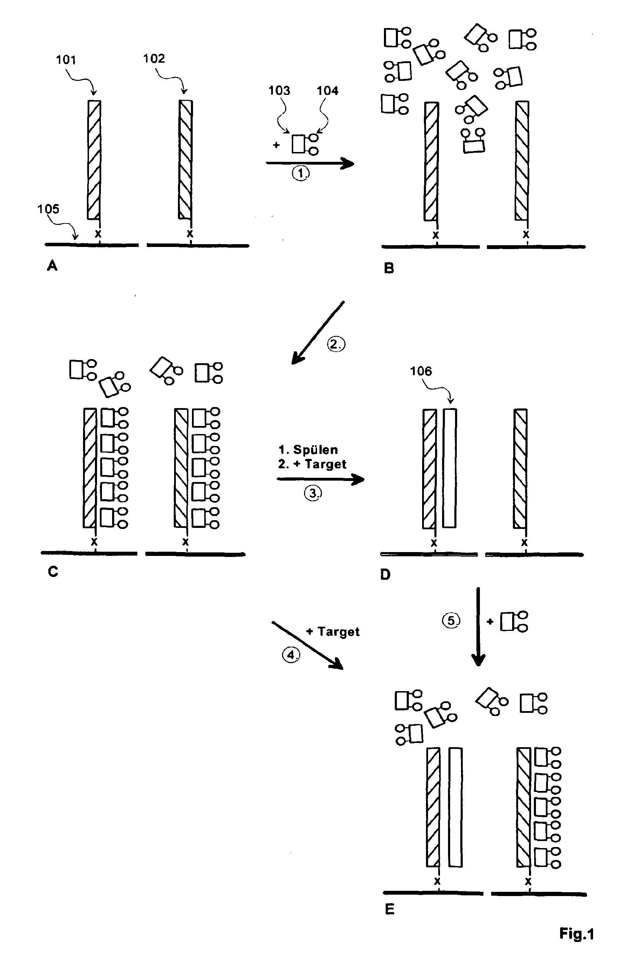

Nach der Hybridisierung zwischen Ligat und Ligand wird in einer zweiten Messung (z.B. einer zweiten chronocoulometrischen Messung, vgl. Abschnitt "Oberflächensensitive Detektionsmethoden") der Anteil der verbliebenen oberflächenimmobilisierten Signal-Nukleinsäureoligomer-Liganden bestimmt. Die Differenz aus Referenzmessung und zweiter Messung je Test-Site ist proportional zur Anzahl der ursprünglich in der Untersuchungslösung für das jeweilige Test-Site vorhandenen komplementären (bzw. in weiten Bereichen komplementären) Ligand-Oligonukleotide (vgl. Fig. 1, Verfahrensweg mit den Schritten ①, ②, ④).

Alternativ können nach der Referenzmessung die Assoziate aus Ligat-Nukleinsäureoligomer und Signal- Nukleinsäureoligomer-Ligand an der Oberfläche, z.B. durch Temperaturerhöhung, dissoziiert und sämtliche Signal-Nukleinsäureoligomer-Liganden oder nur die Signal-Nukleinsäureoligomer-Liganden in der überstehenden Lösung durch Waschen entfernt werden, so dass nach der Referenzmessung die ursprünglich eingesetzte Oberfläche mit immobilisierten Ligat-Oligonukleotiden zur Verfügung steht. Das Entfernen der Signal-Nukleinsäureoligomer-Liganden aus den Assoziaten mit den Ligat-Nukleinsäureoligomeren erfolgt im allgemeinen durch Herausnehmen der modifizierten Oberfläche aus der die Signal-Nukleinsäureoligomer-Liganden enthaltenden Lösung und anschließendem Waschen der modifizierten Oberfläche. Dehybridisierende Bedingungen können dabei beim Waschen der modifizierten Oberfläche und/oder vor dem Entfernen der modifizierten Oberfläche aus der die Signal-Nukleinsäureoligomer-Liganden enthaltenden Lösung eingestellt werden. Im nächsten Schritt wird dann die Probenlösung mit Ligand-Oligonukleotid(en) zur Oberfläche mit immobilisierten Ligat-Oligonukleotiden gegeben und die möglicherweise vorhandenen Ligand-Oligonukleotide können unter beliebigen, dem Fachmann bekannten Stringenzbedingungen an die Ligat-Oligonukleotide hybridisiert werden. Im Idealfall kann durch das Einstellen der Stringenzbedingungen erreicht werden, dass ausschließlich komplementäre Ligand-Oligonukleotide an den Ligat-Oligonukleotiden hybridisiert bleiben, wohingegen "Ligand-Oligonukleotide", die ein oder mehrere Mismatches aufweisen, dehybridisieren. Überschüssige Untersuchungslösung mit nicht angebundenen Nukleinsäureoligomer-Liganden wird durch Waschen mit geeigneten Pufferlösungen entfernt. Anschließend wird - wie zur Durchführung der Referenzmessung - erneut mit der eine bestimmte Menge an Signal-Nukleinsäureoligomer-Liganden enthaltenden Lösung inkubiert und in der zweiten Messung der Anteil der noch an die Ligat-Oligonukleotide assoziierenden Signal-Nukleinsäureoligomer-Liganden bestimmt (vgl. Fig. 1, Verfahrensweg mit den Schritten ①, ②, ③, ⑤).

**Ausführungsformen**

*a) Kovalente Ausführungsform bei Anbindung von Ligat-Oligonukleotiden an (eine) einzeln adressierbare Gold-Elektrode(n), redoxgelabelten ns-Tetrameren als Signal-Nukleinsäureoligomer-Liganden, Ligand-Oligonukleotiden und chronocoulometrischer Detektion der Verdrängung der Signal-Nukleinsäureoligomer-Liganden durch die Nukleinsäureoligomer-Liganden:*

Die n Nukleotide (nt) lange Ligat-Nukleinsäure (DNA, RNA oder PNA, z.B. ein 20 Nukleotide langes Oligo) (Fig. 1A, 101 bzw. 102) ist in der Nähe eines ihrer Enden (3'-oder 5'-Ende) direkt oder über einen (beliebigen) Spacer mit einer reaktiven Gruppe zur kovalenten Verankerung an der Oberfläche versehen, z.B. als 3'-Thiol-modifiziertes Ligat-Oligonukleotid, bei dem die endständige Thiolmodifikation als reaktive Gruppe zur Anbindung an Goldelektroden dient. Weitere kovalente Verankerungsmöglichkeiten ergeben sich z.B. aus aminomodifiziertem Ligat-Oligonukleotid, das zur Verankerung an oberflächlich zur Carbonsäure aufoxidierten Glaskohlenstoffelektroden oder an Platinelektroden verwendet wird. Zusätzlich kann ein monofunktionaler Linker geeigneter Kettenlänge mit identischer reaktiver Gruppe bereit gestellt werden. Das so modifizierte Ligat-Nukleinsäureoligomer wird

(i) in Puffer (z.B. 50 - 500 mM Phosphat-Puffer, pH = 7, 1mM EDTA) gelöst mit der Oberfläche in Kontakt gebracht und dort über die reaktive Gruppe des Ligat-Nukleinsäureoligomers an die - gegebenenfalls entsprechend derivatisierte - Oberfläche angebunden oder

(ii) in Gegenwart eines monofunktionalen Linkers in Puffer (z.B. 100 mM Phosphat-Puffer, pH = 7, 1mM EDTA, 0,1 - 1 M NaCl) gelöst mit der Oberfläche in Kontakt gebracht und dort über die reaktive Gruppe des Ligat-Nukleinsäureoligomers gemeinsam mit dem monofunktionalen Linker an die - gegebenenfalls entsprechend derivatisierte - Oberfläche angebunden, wobei darauf geachtet wird, dass genügend monofunktionaler Linker geeigneter Kettenlänge zugesetzt wird (etwa 0,1 bis 10 facher oder sogar 100 facher Überschuss), um zwischen den einzelnen Ligat-Oligonukleotiden genügend Freiraum für eine Hybridisierung mit den redoxgelabelten Signal-Nukleinsäureoligomer-Liganden bzw. dem Ligand-Oligonukleotid zur Verfügung zu stellen oder

(iii) in Puffer (z.B. 10 - 350 mM Phosphat-Puffer, pH = 7, 1mM EDTA) gelöst mit der Oberfläche in Kontakt gebracht und dort über die reaktive Gruppe des Ligat-Nukleinsäureoligomers an die - gegebenenfalls entsprechend derivatisierte - Oberfläche angebunden. Anschließend wird die so modifizierte Oberfläche mit dem entsprechenden monofunktionalen Linker in Lösung (z.B. Alkanthiole oder w-Hydroxy-Alkanthiole in Phosphat-Puffer/EtOH-Mischungen bei Thiol-modifizierten Ligat-Oligonukleotiden) in Kontakt gebracht, wobei der monofunktionale Linker über seine reaktive Gruppe an die - gegebenenfalls entsprechend derivatisierte - Oberfläche anbindet (vgl. Abschnitt "Die Oberfläche").

Die so modifizierte Oberfläche (Fig. 1A) wird, nach entsprechenden Waschschritten, mit redoxgelabelten Signal-Nukleinsäureoligomer-Liganden (Fig. 1B, 104) aus weniger als n komplementären Nukleotiden (Fig. 1B, 103) in Kontakt gebracht. Als mit Redoxlabel markierte Signal-Nukleinsäureoligomer-Liganden können z.B. ein- oder mehrfach Ferrocencarbonsäure-modifizierte Nukleinsäure-Tetramere (vgl. Bsp. 3) verwendet werden, deren Sequenz zu Tetramer-Teilsequenzen der Ligat-Oligonukleotide komplementär ist, oder mit Redoxlabel (z.B. Ferrocen-Derivaten) modifiziertes SSB (Single Stranded DNA Binding Protein) eingesetzt werden. Dabei wird darauf geachtet, dass deutlich mehr markierte Signal-Nukleinsäureoligomer-Liganden (mindestens 1.1facher molarer Überschuss) zugegeben werden als über die Ligat-Nukleinsäureoligomere an der Oberfläche gebunden werden können.

Das Detektionslabel am Signal-Nukleinsäureoligomer-Liganden wird durch ein geeignetes Verfahren detektiert, z.B. durch Chronocoulometrie im Fall der mit Ferrocen Redox-Label markierten Signal-Oligonukleotide. Anschließend wird der gelöste Ligand zugegeben und die Messung zur Detektion der Detektionslabel mit dem geeigneten Verfahren wiederholt (z.B. erneute chronocoulometrische Messung im Fall der mit Ferrocen Redox-Label markierten Signal-Oligonukleotide).

Alternativ wird die modifizierte Oberfläche aus der Signal-Nukleinsäureoligomer-Liganden enthaltenden Lösung entfernt, gegebenenfalls wie oben beschrieben gewaschen und anschließend mit der die Nukleinsäureoligomer-Liganden enthaltenden Lösung in Kontakt gebracht. Die Hybridisierung kann unter geeigneten, dem Fachmann bekannten Bedingungen durchgeführt werden (beliebige, frei wählbare Stringenzbedingungen der Parameter Potential/Temperatur/Salz/chaotrope Salze etc. für die Hybridisierung). Danach wird die modifizierte Oberfläche mit Signal-Nukleinsäureoligomer-Liganden (in gleicher Konzentration wie bei der vorangegangenen Messung) in Kontakt gebracht und eine Messung zur Detektion der Detektions-Label mit einem geeigneten Verfahren durchgeführt.

Der Unterschied im Messsignal (Ab- bzw. Zunahme, je nach Messverfahren) ist proportional zur Anzahl der Hybridisierungsereignisse zwischen Ligat-Nukleinsäureoligomer auf der Oberfläche und passendem Nukleinsäureoligomer-Liganden in der Untersuchungslösung (vgl. Bsp. 6). Bei der chronocoulometrischen Messung wird eine Abnahme des nahezu instantanen Signalanteils des Oberflächenüberschusses an Redoxlabeln detektiert, vgl. "Oberflächensensitive Detektionsmethoden".

Als Variante des beschriebenen Verfahrens werden die Komplexe aus oberflächengebundenem Ligat-Nukleinsäureoligomeren und markierten Signal-Nukleinsäureoligomer-Liganden (Fig. 1C) nach erfolgter erster Detektion (Referenzmessung) gelöst (z.B. durch Temperaturerhöhung) und die freien markierten Signal-Nukleinsäureoligomer-Liganden durch Waschen aus dem Überstand entfernt (Fig. 1, ③). Nach Zugabe des Nukleinsäureoligomer-Liganden, Hybridisierung (Fig. 1D) und Zugabe der markierten Signal-Nukleinsäureoligomer-Liganden (Fig. 1, ⑤) wird eine zweite chronocoulometrische Messung (Fig. 1 E) durchgeführt.

Das Verfahren kann für eine Ligand-Art, also eine bestimmte Ligand-Oligonukleotid-Art mit bekannter Sequenz, an einer Elektrode oder für mehrere Ligand-Arten, also verschiedene Ligand-Oligonukleotid-Arten, an einzeln adressierbaren Elektroden eines Elektrodenarrays, das bei komplexeren Arrays z.B. über CMOS-Technologie ansteuer- und auslesbar ist, angewendet werden.

b) *SNP-Ausführungsform bei Anbindung von **20** Nukleotide umfassenden Ligat-Oligonukleotiden an (eine) einzeln adressierbare Gold-Elektrode(n), redoxgelabelten SNP-ID-Liganden aus **19** Nukleotiden, deren Sequenz komplementär zur Sequenz der Ligat-Nukleinsäureoligomere ist, Ligand-Oligonukleotiden und cyclovoltammetrischer Detektion der Signal-Nukleinsäureoligomer-Liganden:*

Die 20 Nukleotide (nt) lange Ligat-Nukleinsäure (DNA, RNA oder PNA, Fig. 1A, 101 bzw. 102) ist in der Nähe eines ihrer Enden (3'- oder 5'-Ende) direkt oder über einen (beliebigen) Spacer mit einer reaktiven Gruppe zur kovalenten Verankerung an der Oberfläche versehen, z.B. als 3'-Thiol-modifiziertes Ligat-Oligonukleotid, bei dem die endständige Thiolmodifikation als reaktive Gruppe zur Anbindung an Goldelektroden dient. Das so modifizierte Ligat-Nukleinsäureoligomer wird

(i) in Puffer (z.B. 50 - 500 mM Phosphat-Puffer, pH = 7, 1mM EDTA) gelöst mit der Oberfläche in Kontakt gebracht und dort über die reaktive Gruppe des Ligat-Nukleinsäureoligomers an die - gegebenenfalls entsprechend derivatisierte - Oberfläche angebunden oder

(ii) in Gegenwart eines monofunktionalen Linkers in Puffer (z.B. 100 mM Phosphat-Puffer, pH = 7, 1mM EDTA, 0,1 - 1 M NaCl) gelöst mit der Oberfläche in Kontakt gebracht und dort über die reaktive Gruppe des Ligat-Nukleinsäureoligomers gemeinsam mit dem monofunktionalen Linker an die - gegebenenfalls entsprechend derivatisierte - Oberfläche angebunden, wobei darauf geachtet wird, dass genügend monofunktionaler Linker geeigneter Kettenlänge zugesetzt wird (etwa 0,1 bis 10 facher oder sogar 100 facher Überschuss), um zwischen den einzelnen Ligat-Oligonukleotiden genügend Freiraum für eine Hybridisierung mit den redoxgelabelten Signal-Nukleinsäureoligomer-Liganden bzw. dem Ligand-Oligonukleotid zur Verfügung zu stellen oder

(iii) in Puffer (z.B. 10 - 350 mM Phosphat-Puffer, pH = 7, 1mM EDTA) gelöst mit der Oberfläche in Kontakt gebracht und dort über die reaktive Gruppe des Ligat-Nukleinsäureoligomers an die - gegebenenfalls entsprechend derivatisierte - Oberfläche angebunden. Anschließend wird die so modifizierte Oberfläche mit dem entsprechenden monofunktionalen Linker in Lösung (z.B. Alkanthiole oder ω-Hydroxy-Alkanthiole in Phosphat-Puffer/EtOH-Mischungen bei Thiol-modifizierten Ligat-Oligonukleotiden) in Kontakt gebracht, wobei der monofunktionale Linker über seine reaktive Gruppe an die - gegebenenfalls entsprechend derivatisierte - Oberfläche anbindet (vgl. Abschnitt "Die Oberfläche").

Die so modifizierte Oberfläche (Fig. 1A) wird, nach entsprechenden Waschschritten, mit den Nukleinsäureliganden in Kontakt gebrach. Nach 30 min. Reaktionszeit wird die modifizierte Oberfläche mit Puffer (z.B. 350 mM Phosphat-Puffer, pH = 7, 1mM EDTA) gespült, um nicht an Ligat-Nukleinsäureoligomere assoziierte Nukleinsäureoligomere von der Oberfläche und der überstehenden Lösung zu entfernen. Anschließend werden redoxgelabelte SNP-ID-Liganden aus 19 Nukleotiden, deren Sequenz zur Sequenz der Ligat-Nukleinsäureoligomere komplimentär ist, mit der Oberfläche in Kontakt gebracht. Als Redoxlabel dient kovalent angebundene Ferrocencarbonsäure. Dabei wird die Menge an SNP-ID-Liganden so eingestellt, dass das molare Verhältnis aus SNP-ID-Liganden und Ligat-Nukleinsäureoligomeren 2 - 5 beträgt und die Konzentration des SNP-ID-Liganden möglichst hoch ist. Nach 30 min. Reaktionszeit wird die modifizierte Oberfläche mit Puffer (z.B. 350 mM Phosphat-Puffer, pH = 7, 1mM EDTA) gespült, um nicht an Ligat-Nukleinsäureoligomere assoziierte SNP-ID-Liganden von der Oberfläche und der überstehenden Lösung zu entfernen.

Das Detektionslabel am SNP-ID-Liganden wird durch ein geeignetes Verfahren detektiert, z.B. durch cyclovoltammetrische Bestimmung der mit Ferrocen Redox-Label markierten Signal-Oligonukleotide. Der erhaltene Wert wird mit einem vorher ermittelten Referenzwert verglichen, bei dem der SNP-ID-Ligand unter gleichen Hybridisierungsbedingungen (Menge, Konzentration, Hybridisierungsdauer) an eine identische modifizierte Oberfläche, aber in Abwesenheit von Nukleinsäureoligomer-Liganden, assoziiert wurde.

Bei deutlichem Unterschied des Messsignal vom Referenzwert (mehr als 50 % Abnahme) lag der entsprechende, zum Ligat-Nukleinsäureoligomer komplementäre Nnukleinsäureoligomer-Ligand vor und zwar als perfekter Match für den 20 Nukleotide umfassenden Teilbereich, den der Ligat abdecken kann.

**Beispiel 1**: *Darstellung der N-Hydroxysuccinimid-Aktivester der Redox- (oder Fluorophor-) Label*

1 mmol des jeweiligen Carbonsäurederivates eines Fluorophors (z.B. Fluorescein) bzw. einer redoxaktiven Substanz (z.B. Ferrocen) und 1.1 mmol N-Hydroxysuccinimid werden in 15 ml wasserfreiem Dioxan gelöst. 1.1 mmol Carbodiimid (gelöst in 3 ml wasserfreiem Dioxan) werden unter Eiskühlung zum Carbonsäurederivat getropft. Das Reaktionsgemisch wird 16 h bei RT gerührt, der gebildete Niederschlag abfiltriert und das Lösungsmittel abgezogen. Der Rückstand wird durch Kieselgelchromatographie aufgereinigt (Merck Kieselgel 60, Laufmittel: Dichlormethan/Ethylacetat/Heptan-Mischungen).

**Beispiel 2**: *Darstellung der aminomodifizierten Oligonukleotide zur Kopplung der Aktivesterlabel aus Bsp. 1 bzw. von thiolmodifizierten Oligonukleotiden zur Verankerung auf Gold als Ligat-Nukleinsäureoligomere*

Die Synthese der Oligonukleotide erfolgt in einem automatischen Oligonukleotid-Synthesizer (Expedite 8909; ABI 384 DNA/RNA-Synthesizer) gemäß der vom Hersteller empfohlenen Syntheseprotokolle für eine 1.0 $\mu$mol Synthese.
Die Synthese der (Signal-) Nukleinsäureoligomer-Liganden erfolgt standardmäßig an A-CPG als Trägermaterial. Modifikationen an der 5'-Position der Oligonukleotide erfolgen mit einem auf 5 Minuten verlängerten Kopplungsschritt. Der Amino-Modifier C2 dT (Glen Research 10-1037) wird in die Sequenzen mit dem jeweiligen Standardprotokoll eingebaut.
Die Darstellung von 3'-thiolmodifizierten Ligat-Oligonukleotiden (bzw. $HO-(CH_2)_2-SS-(CH_2)_2OPO_3$-Oligonukleotiden) erfolgt an 1-O-Dimethoxytrityl-propyl-disulfid-CPG-Träger (Glen Research 20-2933) analog zu Standardprotokollen, wobei die Oxidationsschritte mit einer 0.02 M Iodlösung durchgeführt werden, um eine oxidative Spaltung der Disulfidbrücke zu vermeiden.
Die Kopplungseffizienzen werden während der Synthese online über die DMT-Kationen-Konzentration photometrisch bzw. konduktometrisch bestimmt.
Die Oligonukleotide werden mit konzentriertem Ammoniak (30%) bei 37 °C über einen Zeitraum von 16 h entschützt. Die Reinigung der Oligonukleotide erfolgt mittels RP-HPL Chromatographie nach Standardprotokollen (Laufmittel: 0.1 M Triethylammoniumacetat-Puffer, Acetonitril), die Charakterisierung mittels MALDI-TOF MS.

**Beispiel 3**: *Umsetzung der aminomodifizierten Oligonukleotide (Bsp. 2) mit den N-Hydroxy-Aktivestern (Bsp. 1)*

Die aminomodifizierten Oligonukleotide werden in 0.1 M Boratpuffer (pH 8.5) gelöst und mit den in DMSO gelösten N-Hydroxysuccinimid-Aktivestern gemäß dem Protokoll von Molecular Probes (Labeling Amine-Modified Oligonucleotides) umgesetzt. Die Reinigung der Oligonukleotide erfolgt mittels RP-HPL Chromatographie nach Standardprotokollen (Laufmittel: 0.1 M Triethylammoniumacetat-Puffer, Acetonitril), die Charakterisierung mittels MALDI-TOF MS.

**Beispiel 4**: *Herstellung der Oligonukleotid-Elektrode Au-S(CH$_2$)$_2$-ss-oligo*

Die Herstellung von Au-S(CH$_2$)$_2$-ss-oligo gliedert sich in 2 Teilabschnitte, nämlich der Darstellung der leitfähigen Oberfläche und der Derivatisierung der Oberfläche mit dem Ligat-Oligonukleotid in Gegenwart eines geeigneten monofunktionalen Linkers (Inkubationsschritt).
Das Trägermaterial für die kovalente Anbindung der Doppelstrang-Oligonukleotide bildet ein ca. 100 nm dünner Gold-Film auf Mica (Muskovit Plättchen). Dazu wird in einer elektrischen Entladungskammer frisch gespaltenes Mica mit einem Argon-Ionenplasma gereinigt und durch elektrische Entladung Gold (99.99%) in einer Schichtdicke von ca. 100 nm aufgebracht. Anschließend wird der Gold-Film mit 30% $H_2O_2$ / 70% $H_2SO_4$ von Oberflächenverunreinigungen befreit (Oxidation organischer Ablagerungen) und für ca. 20 Minuten in Ethanol getaucht, um an der Oberfläche adsorbierten Sauerstoff zu verdrängen. Nach Abspülen der Oberfläche mit bidestilliertem Wasser wird auf die horizontal gelagerte Oberfläche eine vorher bereitete 1x10$^{-4}$ molare Lösung des (modifizierten) Oligonukleotids aufgetragen, so dass die komplette Gold-Oberfläche benetzt wird (Inkubationsschritt, siehe auch unten).
Zur Inkubation wird ein modifiziertes 20 bp Einzelstrang-Oligonukleotid der Sequenz 5'-TAG CGG ATA ACA CAG TCA CC-3' verwendet, das an der Phosphatgruppe des 3' Endes mit $(HO-(CH_2)_2-S)_2$ zum $P-O-(CH_2)_2-S-S-(CH_2)_2-OH$ (vgl. Bsp. 2) verestert ist. Zu einer 5x10$^{-5}$ molaren Lösung dieses Oligonukleotids in HEPES-Puffer (0.1 molar in Wasser, pH 7.5 mit 0.7 molarem Zusatz von TEATFB) wird eine ca. 10$^{-5}$ bis 10$^{-1}$ molare Propanthiol-Lösung (oder ein anderes Thiol oder Disulfid geeigneter Kettenlänge) zugegeben, die Gold-Oberfläche eines Test-Sites komplett benetzt und 2 - 24 h inkubiert. Während dieser Reaktionszeit wird der Disulfidspacer $P-O-(CH_2)_2-S-S-(CH_2)_2-OH$ des Oligonukleotids homolytisch gespalten. Dabei bildet der Spacer mit Au-Atomen der Oberfläche eine kovalente Au-S-Bindung aus, wodurch es zu einer 1:1 Koadsorption des ss-Oligonukleotids und des abgespaltenen 2-Hydroxy-mercaptoethanols kommt. Das in der Inkubationslösung gleichzeitig anwesende, freie Propanthiol wird ebenfalls durch Ausbildung einer Au-S-Bindung koadsorbiert (Inkubationsschritt). Statt des Einzelstrang-Oligonukleotids kann dieser Einzelstrang auch mit seinem unmodifizierten Komplementärstrang hybridisiert sein.

**Beispiel 5**: *Alternative Herstellung der Oligonukleotid-Elektrode Au-S(CH$_2$)$_2$-ss-oligo*

Die alternative Herstellung von Au-S(CH$_2$)$_2$-SS-oligo gliedert sich in 3 Teilabschnitte, nämlich der Darstellung der leitfähigen Oberfläche, der Derivatisierung der Oberfläche mit dem Ligat-Oligonukleotid (Inkubationsschritt) und der Nachbelegung der so modifizierten Elektrode mit einem geeigneten monofunktionalen Linker (Nachbelegungsschritt).

Das Trägermaterial für die kovalente Anbindung der Ligat-Oligonukleotide bildet ein ca. 100 nm dünner Gold-Film auf Mica (Muskovit Plättchen), vgl. Bsp. 4.

Zur Inkubation wird ein modifiziertes 20 bp Einzelstrang-Oligonukleotid der Sequenz 5'-TAG CGG ATA ACA CAG TCA CC-3' verwendet, das an der Phosphatgruppe des 3' Endes mit (HO-(CH$_2$)$_2$-S)$_2$ zum P-O-(CH$_2$)$_2$-S-S-(CH$_2$)$_2$-OH verestert ist. Die Goldoberfläche eines Test-Sites wird mit einer ca. 5x10$^{-5}$ molaren Lösung dieses Oligonukleotids in HEPES-Puffer (0.1 molar in Wasser, pH 7.5) benetzt und 2 - 24 h inkubiert. Während dieser Reaktionszeit wird der Disulfidspacer P-O-(CH$_2$)$_2$-S-S-(CH$_2$)$_2$-OH des Oligonukleotids homolytisch gespalten. Dabei bildet der Spacer mit Au-Atomen der Oberfläche eine kovalente Au-S-Bindung aus, wodurch es zu einer Koadsorption des ss-Oligonukleotids und des abgespaltenen 2-Hydroxy-mercaptoethanols kommt (Inkubationsschritt).

Anschließend wird die so modifizierte Gold-Elektrode mit einer ca. 10$^{-5}$ bis 10$^{-1}$ molaren Propanthiol-Lösung (in Wasser oder Puffer, pH 7 - 7.5) oder mit einem anderen Thiol oder Disulfid (geeigneter Kettenlänge) komplett benetzt und 2 - 24h inkubiert. Das freie Propanthiol belegt nach dem Inkubationsschritt verbleibende freie Gold-Oberfläche durch Ausbildung einer Au-S-Bindung.

**Beispiel 6:** *Chronocoulometrische Messung am System Au-ss-oligo/Ferrocen-modifizierte Nukleinsäure-Tetramere in Abwesenheit und Gegenwart von Nukleinsäureoligomer-Liganden (komplementär zu ss-oligo in Au-ss-oligo)*

Gemäß Bsp. 5 wird eine Sonden-Elektrode hergestellt. Dazu wird das oben beschriebene HO-(CH$_2$)$_2$-SS-(CH$_2$)$_2$-modifizierte Oligonukleotid (Sequenz TAG CGG ATA ACA CAG TCA CC) auf Gold immobilisiert (50 μmol Oligonukleotid in Phosphat-Puffer (500 mM K$_2$HPO$_4$/KH$_2$PO$_4$ pH 7), Nachbelegung mit 1 mM Propanthiol in Wasser).

Nach Zugabe von komplementären zweifach ferrocengelabelten Nukleinsäure-Tetrameren (10 μM) wird ein Potentialsprungexperiment durchgeführt. Die durch chronocoulometrische Messung erhaltenen Werte sind in der Figur 3, Kurve 1, dargestellt. Nach Zugabe des komplementären Targets (5 μM) wird das Potentialsprungexperiment wiederholt. Die durch die anschließend wiederholte chronocoulometrische Messung erhaltenen Werte sind in der Figur 3, Kurve 2, dargestellt.

Der Durchmesser der verwendeten Goldelektrode beträgt 6 mm, d.h. für die Immobilisierung der Ligat-Nukleinsäureoligomeren steht eine Fläche von 0.28 cm$^2$ zur Verfügung. Aus den Integralen der Kurven 1 und 2 (Figur 3) ergibt sich eine Differenz von 70 x 10$^{-8}$ C (0,7 μC). Dieser Wert entspricht 2,5 μC/cm$^2$ bzw. 1.6 x 10$^{13}$ Elektronen/cm$^2$. Bei Annahme einer maximalen Belegung mit Ligat-Oligonukleotiden, also einer Belegung mit 7 x 10$^{12}$ Ligat-Oligonukleotiden pro cm$^2$, wurden somit im Mittel mindestens ca. 2.2 Elektronen pro Ligat-Oligonukleotid umgesetzt, d.h. 2.2 Ferrocenlabel durch Hybridisierung des Ligat-Nukleinsäureoligomeren mit dem Nukleinsäureoligomer-Liganden verdrängt. Im Mittel sind somit 1.1 Tetramere von dem Ligat-Oligonukleotid verdrängt worden.

**Patentansprüche**

1. Verfahren zur Detektion von Nukleinsäureoligomer-Hybridisierungsereignissen umfassend die Schritte

a) Bereitstellen einer modifizierten leitfähigen Oberfläche, wobei die Modifikation in der Anbindung von wenigstens zwei Arten von Ligat-Nukleinsäureoligomeren besteht, wobei die verschiedenen Arten von Ligat-Nukleinsäureoligomeren in räumlich abgetrennten Bereichen an die Oberfläche gebunden sind,
b) Bereitstellen von mit einem Detektionslabel modifizierten Signal-Nukleinsäureoligomer-Liganden, wobei als Detektionslabel eine redoxaktive Substanz verwendet wird,

c) Bereitstellen einer Probe mit Nukleinsäureoligomer-Liganden,

d) Inkontaktbringen einer definierten Menge der Signal-Nukleinsäureoligomer-Liganden mit der modifizierten Oberfläche und Inkontaktbringen der Probe und der darin enthaltenen Nukleinsäureoligomer-Liganden mit der modifizierten Oberfläche, wobei zunächst der Schritt

d$_1$) Inkontaktbringen einer definierten Menge der Signal-Nukleinsäureoligomer-Liganden mit der modifizierten Oberfläche,
danach der Schritt
d$_3$) Detektion der Signal-Nukleinsäureoligomer-Liganden und danach der Schritt
d$_2$) Inkontaktbringen der Probe mit der modifizierten Oberfläche durchgeführt wird,

e) Detektion der Signal-Nukleinsäureoligomer-Liganden durch eine oberflächerisensitive elektrochemische Methode,
f) Vergleich der in Schritt e) erhaltenen Werte mit den in Schritt d$_3$) erhaltenen Referenzwerten.

2. Verfahren nach Anspruch 1, wobei nach Schritt d$_3$) und vor Schritt d$_2$) der Schritt

d$_4$) Waschen der modifizierten Oberfläche

durchgeführt wird und nach Schritt d$_2$) und vor Schritt e) der Schritt

d$_5$) Inkontaktbringen der Signal-Nukleinsäureoligomer-Liganden mit der modifizierten Oberfläche, wobei die gleiche definierte Menge an Signal-Nukleinsäureoligomer-Liganden wie in Schritt d$_1$) verwendet wird

durchgeführt wird.

3. Verfahren nach Anspruch 2, wobei nach Schritt d$_3$) und vor oder während Schritt d$_4$) der Schritt

d$_6$) Einstellen von Bedingungen oder Ergreifen von Maßnahmen, die zur zumindest überwiegenden Dissoziation von Ligat-Nukleinsäureoligomeren und Signal-Nukleinsäureoligomer-Liganden führen

durchgeführt wird.

4. Verfahren nach Anspruch 3, wobei in Schritt d$_6$) die Temperatur über die Schmelztemperatur der aus Ligat-Nukleinsäureoligomer und Signal-Nukleinsäureoligomer-Ligand bestehenden doppelsträngigen Oligonukleotide erhöht wird.

5. Verfahren nach Anspruch 3, wobei in Schritt d$_6$) chaotrope Salze zugesetzt werden.

6. Verfahren nach Anspruch 3, wobei in Schritt d$_6$) ein Potential angelegt wird, das über dem elektrostringenten Potential liegt.

7. Verfahren nach Anspruch 1, wobei nach Schritt e) die Schritte

e$_1$) Einstellen von Bedingungen oder Ergreifen von Maßnahmen, die zur zumindest überwiegenden Dissoziation von Ligat-Nukleinsäureoligomeren und Nukleinsäureoligomer-Liganden und zur zumindest überwiegenden Dissoziation von Ligat-Nukleinsäureoligomeren und Signal-Nukleinsäureoligomer-Liganden führen, Waschen der modifizierten Oberfläche,
e$_2$) Inkontaktbringen der Signal-Nukleinsäureoligomer-Liganden mit der modifizierten Oberfläche, wobei die gleiche definierte Menge an Signal-Nukleinsäureoligomer-Liganden wie in Schritt d) verwendet wird,
e$_3$) Detektion der Signal-Nukleinsäureoligomer-Liganden

durchgeführt werden und in Schritt f) die in Schritt e) erhaltenen Werte mit den in Schritt e$_3$) erhaltenen Referenzwerten verglichen werden.

8. Verfahren nach Anspruch 7, wobei in Schritt e$_1$) die Temperatur über die Schmelztemperatur der aus Ligat-Nukleinsäureoligomeren und Nukleinsäureoligomer-Liganden bestehenden doppelsträngigen Oligonukleotide und über die Schmelztemperatur der aus Ligat-Nukleinsäureoligomeren und Signal-Nukleinsäureoligomer-Liganden bestehenden doppelsträngigen Oligonukleotide erhöht wird.

9. Verfahren nach Anspruch 7, wobei in Schritt $e_1$) chaotrope Salze zugesetzt werden.

10. Verfahren nach Anspruch 7, wobei in Schritt $e_1$) ein Potential angelegt wird, das über dem elektrostringenten Potential liegt.

11. Verfahren nach einem der Ansprüche 7 bis 10, wobei vor Schritt $e_3$) der Schritt

   $e_4$) Waschen der modifizierten Oberfläche

durchgeführt wird.

12. Verfahren nach einem der vorhergehenden Ansprüche, wobei als Schritt a) der Schritt

   a) Bereitstellen einer modifizierten Oberfläche, wobei die Modifikation in der Anbindung von wenigstens zwei Arten von Ligat-Nukleinsäureoligomeren besteht und die verschiedenen Arten von Ligat-Nukleinsäureoligomeren in räumlich im wesentlichen abgetrennten Bereichen an die Oberfläche gebunden sind

durchgeführt wird, nach Schritt c) und vor Schritt d) der Schritt

   $c_1$) Zusatz von einer Art von Nukleinsäureoligomer-Liganden zu der Probe, wobei der Nukleinsäureoligomer-Ligand ein Bindungspartner mit hoher Assoziationskonstante einer in einem bestimmten Bereich $T_{100}$ an die Oberfläche gebundenen Art von Ligat-Nukleinsäureoligomer ist, wobei der Nukleinsäureoligomer-Ligand in einer Menge zugegeben wird, die größer ist als die Menge an Nukleinsäureoligomer-Liganden, die notwendig ist, um die Ligat-Nukleinsäureoligomere des $T_{100}$-Test-Sites vollständig zu assoziieren

durchgeführt wird und in Schritt f) die in Schritt e) erhaltenen Werte mit dem für den Bereich $T_{100}$ erhaltenen Wert verglichen werden.

13. Verfahren nach Anspruch 12, wobei als Schritt a) der Schritt

   a) Bereitstellen einer modifizierten Oberfläche, wobei die Modifikation in der Anbindung von wenigstens drei Arten von Ligat-Nukleinsäureoligomeren besteht und die verschiedenen Arten von Ligat-Nukleinsäureoligomeren in räumlich im wesentlichen abgetrennten Bereichen an die Oberfläche gebunden sind, wobei wenigstens eine Art von Ligat-Nukleinsäureoligomeren in einem bestimmten Bereich $T_0$ an die Oberfläche angebunden ist, und wobei in der Probe kein Bindungspartner mit hoher Assoziationskonstante zu dieser Art von Ligat-Nukleinsäureoligomer enthalten ist

durchgeführt wird und in Schritt f) die in Schritt e) erhaltenen Werte mit dem für den Bereich $T_{100}$ erhaltenen Wert und mit dem für den Bereich $T_0$ erhaltenen Wert verglichen werden.

14. Verfahren nach Anspruch 12 oder 13, wobei vor Schritt d) und nach Schritt $c_1$) der Schritt

   $c_2$) Zusatz von wenigstens einer weiteren Art von Nukleinsäureoligomer-Liganden zu der Probe, wobei der Nukleinsäureoligomer-Ligand in der in Schritt c) bereitgestellten Probe nicht enthalten ist und der Nukleinsäureoligomer-Ligand eine Assoziationskonstante $>0$ zu einer in einem bestimmten Bereich $T_n$ an die Oberfläche gebundenen Art von Ligat-Nukleinsäureoligomeren aufweist, wobei der Nukleinsäureoligomer-Ligand in einer Menge zugegeben wird, dass nach Schritt d) n% der Ligat-Nukleinsäureoligomere in dem Bereich $T_n$ in assoziierte Form vorliegen

durchgeführt wird und in Schritt f) die in Schritt e) erhaltenen Werte mit dem für den Bereich $D_{100}$ erhaltenen Wert, mit dem für den Bereich $T_0$ erhaltenen Wert und mit den für die Bereiche $T_n$ erhaltenen Werten verglichen werden.

15. Verfahren nach einem der Ansprüche 12 bis 14, wobei die Signal-Nukleinsäureoligomer-Liganden in einer Menge zugegeben werden, die größer ist als die Menge an Signal-Nukleinsäureoligomer-Liganden, die notwendig ist, um die Ligat-Nukleinsäureoligomere der $T_{100}$-Test-Sites vollständig zu assoziieren.

**16.** Verfahren nach einem der Ansprüche 1 bis 15, wobei die Signal-Nukleinsäureoligomer-Liganden mit mehreren Detektionslabel modifiziert sind.

**17.** Verfahren nach einem der Ansprüche 1 bis 16, wobei die elektrochemische Detektion durch Amperometrie, Chronocoulometrie, Impedanzmessung oder Scanning Electrochemical Microscopy (SECM) erfolgt.

**18.** Verfahren nach einem der vorhergehenden Ansprüche, wobei die als Ligat-Nukleinsäureoligomere verwendeten Nukleinsäureoligomere 3 bis 80 Nukleinsäuren, insbesondere 5 bis 50 Nukleinsäuren, besonders bevorzugt 15 bis 35 oder 8 bis 25 Nukleinsäuren umfassen.

**19.** Verfahren nach einem der vorhergehenden Ansprüche, wobei Signal-Nukleinsäureoligomer-Liganden verwendet werden, die aus n oder mehr Nukleotiden bestehen und nur solche Bereiche aus n Nukleotiden aufweisen, deren Sequenz in weniger als n Nukleotiden, insbesondere in n-1, n-2, n-3, n-4 oder n-5 Nukleotiden zur Sequenz des Ligat-Nukleinsäureoligomers komplementär ist, wobei das Ligat-Nukleinsäureoligomer n Nukleotide umfasst, und wobei n eine ganze Zahl von 6 bis 80, insbesondere von 6 bis 50, besonders bevorzugt von 15 bis 35 oder von 8 bis 25 ist.

**20.** Verfahren nach einem der Ansprüche 1 bis 18, wobei jede Art von Signal-Nukleinsäureoligomer-Ligand wenigstens einen Sequenzabschnitt aufweist, der aus maximal n-1, n-2, n-3, n-4 oder n-5 Nukleotiden besteht und der zu einem Sequenzabschnitt des Ligat-Nukleinsäureoligomers komplementär ist, wobei das Ligat-Nukleinsäureoligomer n Nukleotide umfasst, und wobei n eine ganze Zahl von 6 bis 80, insbesondere von 6 bis 50, besonders bevorzugt von 15 bis 35 oder von 8 bis 25 ist.

**21.** Verfahren nach einem der Ansprüche 1 bis 18, wobei jede Art von Signal-Nukleinsäureoligomer-Ligand mehrere Teilsequenzabschnitte aufweist, wobei die mehreren Teilsequenzabschnitte in der Summe aus insgesamt n-1, n-2, n-3, n-4 oder n-5 Nukleotiden bestehen und die mehreren Teilsequenzabschnitte zu einem Sequenzabschnitt des Ligat-Nukleinsäureoligomers komplementär ist, wobei das Ligat-Nukleinsäureoligomer n Nukleotide umfasst, und wobei n eine ganze Zahl von 6 bis 80, insbesondere von 6 bis 50, besonders bevorzugt von 15 bis 35 oder von 8 bis 25 ist.

**22.** Verfahren nach einem der Ansprüche 1 bis 18, wobei jeweils eine Art von Ligat-Nukleinsäureoligomeren aus einer Anzahl n Nukleotiden besteht und die zu dieser Art von Ligat-Nukleinsäureoligomeren komplementären Signal-Nukleinsäureoligomer-Liganden aus einer Anzahl von n-1, n-2, n-3, n-4 oder n-5 Nukleotiden bestehen, wobei n eine ganze Zahl von 6 bis 80, insbesondere von 6 bis 50, besonders bevorzugt von 15 bis 35 oder von 8 bis 25 ist.

**23.** Verfahren nach einem der Ansprüche 19 bis 22, wobei jeweils eine Art von Nukleinsäureoligomer-Liganden ein Nukleotid aufweist, das nicht komplementär zu dem entsprechenden Nukleotid der komplementären Art von Ligat-Nukleinsäureoligomer ist, und wobei alle anderen Nukleotide besagter Art von Nukleinsäureoligomer-Ligand komplementär zu den entsprechenden Nukleotiden der komplementären Art von Ligat-Nukleinsäureoligomer sind.

**24.** Verfahren nach einem der Ansprüche 19 bis 23, wobei vor Schritt $d_3$) der Schritt

$d_7$) Waschen der modifizierten Oberfläche

durchgeführt wird.

**25.** Verfahren nach einem der Ansprüche 19 bis 23, wobei vor Schritt $e_3$) der Schritt

$e_4$) Waschen der modifizierten Oberfläche

durchgeführt wird.

**26.** Verfahren nach einem der Ansprüche 19 bis 25, wobei vor Schritt e) der Schritt

$d_8$) Waschen der modifizierten Oberfläche

durchgeführt wird.

**EP 1 554 396 B1**

**27.** Verfahren nach einem der Ansprüche 12 bis 26, wobei die verschiedenen Arten von Ligat-Nukleinsäureoligomeren jeweils eine unterschiedliche Anzahl an Nukleinsäuren aufweisen.

**28.** Verfahren nach einem der vorhergehenden Ansprüche, wobei verschieden Arten von Signal-Nukleinsäureoligomer-Liganden verwendet werden.

**29.** Verfahren nach Anspruch 28, wobei die verschiedenen Arten von Signal-Nukleinsäureoligomer-Liganden jeweils eine unterschiedliche Anzahl an Nukleotiden aufweisen.

**30.** Verfahren nach einem der vorhergehenden Ansprüche, wobei das molare Verhältnis von Signal-Nukleinsäureoligomer-Liganden zu Ligat-Nukleinsäureoligomeren zwischen 0,01 und 1000, bevorzugt zwischen 0,1 und 100, besonders bevorzugt zwischen 1 und 10 liegt.

**31.** Verfahren nach einem der vorhergehenden Ansprüche, wobei als Signal-Nukleinsäureoligomer-Liganden Signal-PNA-Oligomer-Liganden verwendet werden.

**Claims**

**1.** A method for detection of nucleic acid oligomer hybridization events, comprising the steps

a) providing a modified conductive surface, the modification consisting in the attachment of at least two types of ligate nucleic acid oligomers, and the differing types of ligate nucleic acid oligomers being bound to the surface in spatially separate regions
b) providing signal nucleic acid oligomer ligands modified with a detection label, wherein a redox-active substance is used as the detection label,
c) providing a sample having nucleic acid oligomer ligands,
d) bringing a defined quantity of the signal nucleic acid oligomer ligands into contact with the modified surface and bringing the sample and the nucleic acid oligomer ligands contained therein into contact with the modified surface, wherein first the step

$d_1$) bringing a defined quantity of the signal nucleic acid oligomer ligands into contact with the modified surface
thereafter the step
$d_3$) detecting the signal nucleic acid oligomer ligands and thereafter the step
$d_2$) bringing the sample into contact with the modified surface is carried out

e) detecting the signal nucleic acid oligomer ligands by a surface-sensitive electrochemical method,
f) comparing the values obtained in step e) with reference values obtained in step $d_3$).

**2.** The method according to claim 1, wherein after step $d_3$) and before step $d_2$) the step

$d_4$) washing the modified surface

is carried out, and after step $d_2$) and before step e) the step

$d_5$) bringing the signal nucleic acid oligomer ligands into contact with the modified surface, the identical defined quantity of signal nucleic acid oligomer ligands being used as in step $d_1$)

is carried out.

**3.** The method according to claim 2, wherein after step $d_3$) and before or during step $d_4$) the step

$d_6$) setting conditions or taking actions that lead to at least predominant dissociation of ligate nucleic acid oligomers and signal nucleic acid oligomer ligands

is carried out.

26

**4.** The method according to claim 3, wherein in step $d_6$), the temperature is raised above the melting temperature of the double-stranded oligonucleotides consisting of ligate nucleic acid oligomer and signal nucleic acid oligomer ligand.

**5.** The method according to claim 3, wherein in step $d_6$), chaotropic salts are added.

**6.** The method according to claim 3, wherein in step $d_6$), a potential that lies above the electrostringent potential is applied.

**7.** The method according to claim 1, wherein after step e) the steps

> $e_1$) setting conditions or taking actions that lead to at least predominant dissociation of ligate nucleic acid oligomers and nucleic acid oligomer ligands, and to at least predominant dissociation of ligate nucleic acid oligomers and signal nucleic acid oligomer ligands, washing the modified surface,
> $e_2$) bringing the signal nucleic acid oligomer ligands into contact with the modified surface, the identical defined quantity of signal nucleic acid oligomer ligands being used as in step d),
> $e_3$) detecting the signal nucleic acid oligomer ligands

> are carried out and in step f), the values obtained in step e) are compared with the reference values obtained in step $e_3$).

**8.** The method according to claim 7, wherein in step $e_1$), the temperature is raised above the melting temperature of the double-stranded oligonucleotides consisting of ligate nucleic acid oligomers and nucleic acid oligomer ligands, and above the melting temperature of the double-stranded oligonucleotides consisting of ligate nucleic acid oligomers and signal nucleic acid oligomer ligands.

**9.** The method according to claim 7, wherein in step $e_1$), chaotropic salts are added.

**10.** The method according to claim 7, wherein in step $e_1$), a potential that lies above the electrostringent potential is applied.

**11.** The method according to one of claims 7 to 10, wherein before step $e_3$) the step

> $e_4$) washing the modified surface

is carried out.

**12.** The method according to one of the preceding claims, wherein as step a) the step

> a) providing a modified surface, the modification consisting in the attachment of at least two types of ligate nucleic acid oligomers, and the differing types of ligate nucleic acid oligomers being bound to the surface in spatially substantially separate regions

is carried out, after step c) and before step d) the step

> $c_1$) adding one type of nucleic acid oligomer ligand to the sample, the nucleic acid oligomer ligand being a binding partner having a high association constant of a type of ligate nucleic acid oligomer that is bound to the surface in a specific region $T_{100}$, the nucleic acid oligomer ligand being added in a quantity that is greater than the quantity of nucleic acid oligomer ligands needed to completely associate the ligate nucleic acid oligomers of the $T_{100}$ test sites

is carried out and in step f), the values obtained in step e) are compared with the value obtained for the $T_{100}$ region.

**13.** The method according to claim 12, wherein as step a) the step

> a) providing a modified surface, the modification consisting in the attachment of at least three types of ligate nucleic acid oligomers, and the differing types of ligate nucleic acid oligomers are bound to the surface in spatially substantially separate regions, at least one type of ligate nucleic acid oligomer being attached to the surface in a specific region $T_0$, and no binding partner having a high association constant to this type of ligate nucleic acid oligomer is contained in the sample

is carried out and in step f), the values obtained in step e) are compared with the value obtained for the $T_{100}$ region, and with the value obtained for the $T_0$ region.

14. The method according to claim 12 or 13, wherein before step d) and after step $c_1$) the step

$c_2$) adding at least one additional type of nucleic acid oligomer ligand to the sample, the nucleic acid oligomer ligand in the sample provided in step c) not being contained, and the nucleic acid oligomer ligand exhibiting an association constant >0 to a type of ligate nucleic acid oligomer that is bound to the surface in a specific region $T_n$, the nucleic acid oligomer ligand being added in a quantity such that, after step d), n% of the ligate nucleic acid oligomers in the $T_n$ region are present in associated form

is carried out and in step f), the values obtained in step e) are compared with the value obtained for the $T_{100}$ region, with the value obtained for the $T_0$ region and with the values obtained for the $T_n$ regions.

15. The method according to one of claims 12 to 14, wherein the signal nucleic acid oligomer ligands are added in a quantity that is greater than the quantity of signal nucleic acid oligomer ligands needed to completely associate the ligate nucleic acid oligomers of the $T_{100}$ test sites.

16. The method according to one of claims 1 to 15, wherein the signal nucleic acid oligomer ligands are modified with multiple detection labels.

17. The method according to one of claims 1 to 16, wherein the electrochemical detection takes place through amperometry, chronocoulometry, impedance measurement or scanning electrochemical microscopy (SECM).

18. The method according to one of the preceding claims, wherein the nucleic acid oligomers used as ligate nucleic acid oligomers comprise 3 to 80 nucleic acids, especially 5 to 50 nucleic acids, particularly preferably 15 to 35 or 8 to 25 nucleic acids.

19. The method according to one of the preceding claims, wherein signal nucleic acid oligomer ligands are used that consist of n or more nucleotides and exhibit only such regions made up of n nucleotides whose sequence is complementary to the sequence of the ligate nucleic acid oligomer in fewer than n nucleotides, especially in n-1, n-2, n-3, n-4 or n-5 nucleotides, the ligate nucleic acid oligomer comprising n nucleotides, and n being an integer from 6 to 80, especially from 6 to 50, particularly preferably from 15 to 35 or from 8 to 25.

20. The method according to one of claims 1 to 18, wherein every type of signal nucleic acid oligomer ligand exhibits at least one sequence section that consists of a maximum of n-1, n-2, n-3, n-4 or n-5 nucleotides and that is complementary to one sequence section of the ligate nucleic acid oligomer, the ligate nucleic acid oligomer comprising n nucleotides, and n being an integer from 6 to 80, especially from 6 to 50, particularly preferably from 15 to 35 or from 8 to 25.

21. The method according to one of claims 1 to 18, wherein every type of signal nucleic acid oligomer ligand exhibits multiple partial-sequence sections, the multiple partial-sequence sections all together consisting of a total of n-1, n-2, n-3, n-4 or n-5 nucleotides, and the multiple partial-sequence sections being complementary to one sequence section of the ligate nucleic acid oligomer, the ligate nucleic acid oligomer comprising n nucleotides, and n being an integer from 6 to 80, especially from 6 to 50, particularly preferably from 15 to 35 or from 8 to 25.

22. The method according to one of claims 1 to 18, wherein, each time, one type of ligate nucleic acid oligomer consists of a number of n nucleotides, and the signal nucleic acid oligomer ligands that are complementary to this type of ligate nucleic acid oligomer consist of a number of n-1, n-2, n-3, n-4 or n-5 nucleotides, n being an integer from 6 to 80, especially from 6 to 50, particularly preferably from 15 to 35 or from 8 to 25.

23. The method according to one of claims 19 to 22, wherein, each time, one type of nucleic acid oligomer ligand exhibits a nucleotide that is not complementary to the corresponding nucleotide of the complementary type of ligate nucleic acid oligomer, and wherein all other nucleotides of said type of nucleic acid oligomer ligand are complementary to the corresponding nucleotides of the complementary type of ligate nucleic acid oligomer.

**24.** The method according to one of claims 19 to 23, wherein before step $d_3$) the step

$d_7$) washing the modified surface

is carried out.

**25.** The method according to one of claims 19 to 23, wherein before step $e_3$) the step

$e_4$) washing the modified surface

is carried out.

**26.** The method according to one of claims 19 to 25, wherein before step e) the step

$d_8$) washing the modified surface

is carried out.

**27.** The method according to one of claims 12 to 26, wherein the differing types of ligate nucleic acid oligomers each exhibit a different number of nucleic acids.

**28.** The method according to one of the preceding claims, wherein differing types of signal nucleic acid oligomer ligands are used.

**29.** The method according to claim 28, wherein the differing types of signal nucleic acid oligomer ligands each exhibit a different number of nucleotides.

**30.** The method according to one of the preceding claims, wherein the molar ratio of signal nucleic acid oligomer ligands to ligate nucleic acid oligomers is between 0.01 and 1000, preferably between 0.1 and 100, particularly preferably between 1 and 10.

**31.** The method according to one of the preceding claims, wherein signal PNA oligomer ligands are used as signal nucleic acid oligomer ligands.

**Revendications**

**1.** Procédé de détection d'événements d'hybridation d'oligomères d'acide nucléique, comprenant les étapes suivantes :

a) préparation d'une surface conductrice modifiée, la modification consistant en la liaison d'au moins deux sortes d'oligomères d'acide nucléique lié, les différentes sortes d'oligomères d'acide nucléique lié étant liées à la surface dans des zones séparées dans l'espace,
b) préparation de ligands d'oligomère d'acide nucléique de signal modifiés par un marquage de détection, une substance rédox active étant utilisée comme marquage de détection,
c) préparation d'un échantillon avec des ligands d'oligomère d'acide nucléique,
d) mise en contact d'une quantité définie des ligands d'oligomère d'acide nucléique de signal avec la surface modifiée et mise en contact de l'échantillon et des ligands d'oligomère d'acide nucléique qu'il contient avec la surface modifiée, dans lequel premièrement l'étape de

d1) mise en contact d'une quantité définie de ligands d'oligomère d'acide nucléique de signal avec la surface modifiées, puis l'étape de
d3) détection des ligands d'oligomère d'acide nucléique de signal et puis l'étape de
d2) mise en contact de l'échantillon avec la surface modifiée est mise en oeuvre,

e) détection des ligands d'oligomère d'acide nucléique de signal à l'aide d'une méthode électrochimique sensible à la surface,
f) comparaison des valeurs obtenues à l'étape e) avec les valeurs de référence obtenues à l'étape d3).

**2.** Procédé selon la revendication 1, dans lequel, après l'étape d3) et avant l'étape d2) l'étape de

d4) lavage de la surface modifiée

est mise en oeuvre et, après l'étape d2) et avant l'étape e), l'étape de

d5) mise en contact des ligands d'oligomère d'acide nucléique de signal avec la surface modifiée, dans laquelle la même quantité définie de ligands d'oligomère d'acide nucléique de signal est utilisée que dans l'étape d1),

est mise en oeuvre.

**3.** Procédé selon la revendication 2, dans lequel, après l'étape d3) et avant ou pendant l'étape d4), l'étape de

d6) ajustement des conditions ou prise des mesures qui conduisent à une dissociation au moins majoritaire des oligomères d'acide nucléique lié et des ligands d'oligomère d'acide nucléique de signal,

est mise en oeuvre.

**4.** Procédé selon la revendication 3, dans lequel, dans l'étape d6), la température est augmentée au-dessus de la température de fusion de l'oligonucléotide à double brin constitué de l'oligomère d'acide nucléique lié et du ligand d'oligomère d'acide nucléique de signal.

**5.** Procédé selon la revendication 3, dans lequel, dans l'étape d6), des sels chaotropiques sont ajoutés.

**6.** Procédé selon la revendication 3, dans lequel, dans l'étape d6), un potentiel situé au-dessus du potentiel de liaison électrique est appliqué.

**7.** Procédé selon la revendication 1, dans lequel, après l'étape e), les étapes

e1) d'ajustement des conditions ou de prise de mesures qui conduisent à la dissociation au moins majoritaire des oligomères d'acide nucléique lié et des ligands d'oligomère d'acide nucléique et à la dissociation au moins majoritaire des oligomères d'acide nucléique lié et des ligands d'oligomère d'acide nucléique de signal, de lavage de la surface modifiée,
e2) de mise en contact des ligands d'oligomère d'acide nucléique de signal avec la surface modifiée, dans laquelle la même quantité définie de ligands d'oligomère d'acide nucléique de signal que dans l'étape d) est utilisée,
e3) détection des ligands d'oligomère d'acide nucléique de signal

sont mises en oeuvre et dans l'étape f), les valeurs obtenues à l'étape e) sont comparées aux valeurs de référence obtenues à l'étape e3).

**8.** Procédé selon la revendication 7, dans lequel, dans l'étape e1), la température est augmentée au-dessus de la température de fusion des oligonucléotides à double brin constitués des oligomères d'acide nucléique lié et des ligands d'oligomère d'acide nucléique et au-dessus de la température de fusion des oligonucléotides à double brin constitués des oligomères d'acide nucléique lié et des ligands d'oligomère d'acide nucléique de signal.

**9.** Procédé selon la revendication 7, dans lequel, dans l'étape e1) des sels chaotropiques sont ajoutés.

**10.** Procédé selon la revendication 7, dans lequel, dans l'étape e1), un potentiel supérieur au potentiel de liaison électrique est appliqué.

**11.** Procédé selon l'une des revendications 7 à 10, dans lequel, dans l'étape e3), l'étape de

e4) lavage de la surface modifiée

est mise en oeuvre.

**12.** Procédé selon l'une des revendications précédentes, dans lequel, en tant qu'étape a) l'étape de

30

a) préparation d'une surface modifiée, dans laquelle la modification consiste en la liaison d'au moins deux sortes d'oligomères d'acide nucléique lié et les différentes sortes d'oligomères d'acide nucléique lié sont liées à la surface dans des zones essentiellement séparées dans l'espace

est mise en oeuvre, après l'étape c) et avant l'étape d), l'étape de

c1) ajout d'une sorte de ligands d'oligomère d'acide nucléique à l'échantillon, le ligand d'oligomère d'acide nucléique étant un partenaire de liaison ayant une constante d'association supérieure d'une sorte d'oligomère d'acide nucléique lié à la surface dans une zone déterminée $T_{100}$, le ligand d'oligomère d'acide nucléique étant ajouté en une quantité qui est supérieure à la quantité de ligands d'oligomère d'acide nucléique qui est nécessaire pour associer totalement les oligomères d'acide nucléique lié du site de test $T_{100}$

est mise en oeuvre et, dans l'étape f), les valeurs obtenues à l'étape e) sont comparées à la valeur obtenue pour la zone $T_{100}$.

13. Procédé selon la revendication 12, dans lequel, en tant qu'étape a), l'étape de

a) préparation d'une surface modifiée, dans laquelle la modification consiste en la liaison d'au moins trois sortes d'oligomères d'acide nucléique lié et les différentes sortes d'oligomères d'acide nucléique lié sont liées à la surface dans des zones essentiellement séparées, au moins une sorte d'oligomères d'acide nucléique lié étant liée à la surface dans une zone déterminée T0, aucun partenaire de liaison ayant une constante d'association supérieure à cette sorte d'oligomères d'acide nucléique lié n'étant contenu dans l'échantillon

est mise en oeuvre, et dans l'étape f) les valeurs obtenues à l'étape e) sont comparées à la valeur obtenue pour la zone $T_{100}$ et avec la valeur obtenue pour la zone $T_0$.

14. Procédé selon la revendication 12 ou 13, dans lequel, avant l'étape d) et après l'étape c1), l'étape de

c2) ajout d'au moins une sorte supplémentaire de ligands d'oligomère d'acide nucléique à l'échantillon, le ligand d'oligomère d'acide nucléique n'étant pas contenu dans l'échantillon préparé à l'étape c) et le ligand d'oligomère d'acide nucléique présentant une constante d'association supérieure à 0 à une sorte d'oligomères d'acide nucléique lié à la surface dans une zone déterminée $T_n$, le ligand d'oligomère d'acide nucléique étant ajouté dans une quantité telle qu'après l'étape d), n% des oligomères d'acide nucléique lié se trouvent sous forme associée dans la zone $T_n$,

est mise en oeuvre et à l'étape f), les valeurs obtenues à l'étape e) sont comparées avec la valeur obtenue pour la zone $T_{100}$, avec la valeur obtenue pour la zone $T_0$ et avec les valeurs obtenues pour les zones $T_n$.

15. Procédé selon l'une des revendications 12 à 14, dans lequel les ligands d'oligomère d'acide nucléique de signal sont ajoutés dans une quantité qui est supérieure à la quantité d'oligomères d'acide nucléique de signal qui est nécessaire pour associer totalement les oligomères d'acide nucléique lié du site test $T_{100}$.

16. Procédé selon l'une des revendications 1 à 15, dans lequel, les ligands d'oligomère d'acide nucléique de signal sont modifiés avec plusieurs marqueurs de détection.

17. Procédé selon l'une des revendications 1 à 16, dans lequel la détection électrochimique s'effectue par ampérométrie, chronocoulométrie, mesure d'impédance ou microscopie électrochimique à balayage (SECM).

18. Procédé selon l'une des revendications précédentes, dans lequel les oligomères d'acide nucléique utilisés comme oligomères d'acide nucléique lié comprennent de 3 à 80 acides nucléiques, en particulier de 5 à 50 acides nucléiques, de façon particulièrement préférée de 15 à 35 ou de 8 à 25 acides nucléiques.

19. Procédé selon l'une des revendications précédentes, dans lequel on utilise des ligands d'oligomère d'acide nucléique de signal qui sont constitués de n ou plus nucléotides et présentent seulement des zones de n nucléotides dont la séquence est complémentaire de moins de n nucléotides, en particulier de n-1, n-2, n-3, n-4 ou n-5 nucléotides à la séquence des oligomères d'acide nucléique lié, l'oligomère d'acide nucléique lié comprenant n nucléotides et n étant un nombre entier allant de 6 à 80, en particulier de 6 à 50, de façon particulièrement préférée de 15 à 35 ou de 8 à 25.

**20.** Procédé selon l'une des revendications 1 à 18, dans lequel chaque sorte de ligand d'oligomère d'acide nucléique de signal présente au moins une section de séquence qui est constituée au maximum de n-1, n-2, n-3, n-4 ou n-5 nucléotides et qui est complémentaire à une section de séquence de l'oligomère d'acide nucléique lié, l'oligomère d'acide nucléique lié comprenant n nucléotides et n étant un nombre de 6 à 80, en particulier de 6 à 50, de façon particulièrement préférée de 15 à 35 ou de 8 à 25.

**21.** Procédé selon l'une des revendications 1 à 18, dans lequel chaque sorte de ligands d'oligomère d'acide nucléique de signal présente plusieurs sections de séquence partielle, les sections de séquence partielle étant constituées ensemble au total de n-1, n-2, n-3, n-4 ou n-5 nucléotides et les plusieurs sections de séquence partielle étant complémetaires à une section de séquence de l'oligomère d'acide nucléique lié, l'oligomère d'acide nucléique lié comprenant n nucléotides et n étant un nombre entier allant de 6 à 80, en particulier de 6 à 50, de façon particulièrement préférée de 15 à 35 ou de 8 à 25.

**22.** Procédé selon l'une des revendications 1 à 18, dans lequel chaque sorte d'oligomères d'acide nucléiques lié est constituée d'un nombre n de nucléotides et les ligands d'oligomères d'acide nucléique de signal complémentaires de cette sorte d'oligomères d'acide nucléique lié sont constitués d'un nombre de n-2, n-3, n-4 ou n-5 nucléotides, n étant un nombre entier allant de 6 à 80, en particulier de 6 à 50, de façon particulièrement préférée de 15 à 35 ou de 8 à 25.

**23.** Procédé selon l'une des revendications 19 à 22, dans lequel chaque sorte de ligands d'oligomères d'acide nucléique présente un nucléotide qui n'est pas complémentaire du nucléotide correspondant de la sorte complémentaire d'oligomère d'acide nucléique lié et dans lequel tous les autres nucléotides de ladite sorte de ligand d'oligomère d'acide nucléique sont complémentaires des nucléotides correspondants de la sorte complémentaire d'oligomères d'acide nucléique lié.

**24.** Procédé selon l'une des revendications 19 à 23, dans lequel, avant l'étape d3), l'étape de

d7) lavage de la surface modifiée

est mise en oeuvre.

**25.** Procédé selon l'une des revendications 19 à 23, dans lequel, avant l'étape e3), l'étape de

e4) lavage de la surface modifiée

est mise en oeuvre.

**26.** Procédé selon l'une des revendications 19 à 25, dans lequel, avant l'étapte e), l'étape de

d8) lavage de la surface modifiée

est mise en oeuvre.

**27.** Procédé selon l'une des revendications 12 à 26, dans lequel les différentes sortes d'oligomères d'acide nucléique lié présentent chacune un nombre différent d'acides nucléiques.

**28.** Procédé selon l'une des revendications précédentes, dans lequel on utilise différentes sortes de ligands d'oligomères d'acide nucléique de signal.

**29.** Procédé selon la revendication 28, dans lequel les différentes sortes de ligands d'oligomères d'acide nucléique de signal présentent chacune un nombre différent de nucléotides.

**30.** Procédé selon l'une des revendications précédentes, dans lequel le rapport molaire des ligands d'oligomère d'acide nucléique de signal sur les oligomères d'acide nucléique lié se situe entre 0,01 et 1000, de préférence entre 0,1 et 100, de façon particulièrement préférée entre 1 et 10.

**31.** Procédé selon l'une des revendications précédentes, dans lequel on utilise comme ligands d'oligomère d'acide nucléique de signal des ligands d'oligomères de PNA de signal.

**Fig.1**

**Fig.2**

**Fig.3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9746568 A
- WO 9951778 A
- WO 0031101 A
- WO 0042217 A
- WO 9321530 A
- WO 9957319 A
- WO 0107665 A2
- US 5753433 A, Kessler
- DE 10141691
- DE 0201269 W

**In der Beschreibung aufgeführte Nicht-Patentliteratur**

- **Wang, J. et al.** *Biosensors & Bioelectronics,* 1997, vol. 12 (7), 587-599
- **Robinson ; Grant.** *Journal of Biological Chemistry,* 1966, vol. 241, 1329ff
- **Steel, A.B. ; Herne, T.M. ; Tarlov M.J.** Electrochemical Quantitation of DNA Immobilized on Gold. *Analytical Chemistry,* 1998, vol. 70, 4670-4677